# EUROPEAN PATENT SPECIFICATION

(11) **EP 2 315 580 B1**
(45) Date of publication and mention of the grant of the patent: **28.12.2011**
(21) Application number: 10711127.0
(22) Date of filing: 26.03.2010
(51) Int. Cl.: A61K 9/00, A61K 47/02, A61K 47/12

(54) **PHARMACEUTICAL FORMULATIONS AND METHODS FOR TREATING RESPIRATORY TRACT INFECTIONS**
PHARMAZEUTISCHE FORMULIERUNGEN UND VERFAHREN ZUR BEHANDLUNG VON ATEMWEGSINFEKTIONEN
COMPOSITIONS PHARMACEUTIQUES ET MÉTHODES DE TRAITEMENT D'INFECTIONS DES VOIES RESPIRATOIRES

(30) Priority: 26.03.2009 US 163772 P; 26.03.2009 US 163763 P; 26.03.2009 US 163767 P; 28.10.2009 US 255764 P; 25.01.2010 US 298092 P; 18.02.2010 US 305819 P
(43) Date of publication of application: 04.05.2011
(73) Proprietor: Pulmatrix, Inc., Lexington, MA 02421 (US)
(72) Inventor: LIPP, Michael M., Framingham Massachusetts 01701 (US); CLARKE, Robert W., Canton Massachusetts 02021 (US); HAVA, David L., Natick Massachusetts 01760 (US); BATYCKY, Richard, Newton Massachusetts 02461 (US); HANRAHAN, John, West Roxbury Massachusetts 02132-2316 (US)
(74) Representative: Lock, Graham James
(86) International application number: PCT/US2010/028906
(87) International publication number: WO 2010/111644

(56) References cited:
- WO-A1-01/76610
- WO-A2-2005/084638
- WO-A2-2006/125153
- US-A- 4 637 815

## Description

### BACKGROUND OF THE INVENTION

Respiratory tract infections are common infections of the upper respiratory tract (e.g., nose, ears, sinuses, and throat) and the lower respiratory tract (e.g., trachea, bronchial tubes, and lungs). Respiratory tract infections may be primary or secondary infections.

Symptoms of upper respiratory tract infections include runny or stuffy nose, irritability, restlessness, poor appetite, decreased activity level, coughing, and fever. Viral infections of the upper respiratory tract cause, or are associated with, for example, sore throats, colds, croup, and the flu. Examples of viruses that cause upper respiratory tract infections include rhinoviruses and influenza viruses. Bacterial infections of the upper respiratory tract cause, or are associated with, for example, whooping cough and strep throat. Exemplary bacteria that cause upper respiratory tract infections include *Streptococcus pyogenes* and *Bordatella pertussis.*

Clinical manifestations of a lower respiratory tract infection include shallow coughing that produces sputum in the lungs, fever, and difficulty breathing. Examples of viral infections of the lower respiratory tract include influenza virus, parainfluenza virus ("PIV") infections, respiratory syncytial virus ("RSV") infections, and bronchiolitis. Examples of bacteria that cause lower respiratory tract infections include *Streptococcus pneumoniae* (which causes pneumonococcal pneumonia) and *Mycobacterium tuberculosis* (which causes tuberculosis).

Respiratory tract infections caused by fungi include systemic candidiasis, blastomycosis crytococcosis, coccidioidomycosis, and aspergillosis.

Influenza, commonly known as flu, is an infectious disease of birds and mammals caused by an RNA virus of the family Orthomyxoviridae (the influenza viruses). Typically, influenza is transmitted from infected mammals through the airborne droplets and aerosols containing the virus, and from infected birds through their droppings. Influenza can also be transmitted by saliva, nasal secretions, feces and blood. Infections occur through contact with these bodily fluids or with contaminated surfaces.

Human parainfluenza viruses (hPIVs) are a group of four distinct serotypes of single-stranded RNA viruses belonging to the paramyxovirus family. They are the second most common cause of lower respiratory tract infection in younger children. Together, the parainfluenza viruses cause ∼75% of the cases of Croup. Repeated infection throughout the life of the host is not uncommon. Symptoms of later breakouts include upper respiratory tract illness as in a cold and sore throat. In immunosuppressed people, such as transplant patients, parainfluenza virus infections can cause severe pneumonia, which sometimes could be fatal.

Human rhinovirus is a genus of the *Picornaviridae* family and is believed to be responsible for between 30% and 50% of common cold infections. Over 100 serotypically distinct strains of human rhinovirus have been identified. No pan-serotype vaccines are available because there is little cross-protection between serotypes. Rhinovirus also plays a significant role in the pathogenesis of otitis media and asthma exacerbations. Infection with rhinovirus leads to the release of inflammatory mediators and increased bronchial responsiveness.

Human respiratory syncytial virus (RSV) is a virus that causes respiratory tract infections. It is the major cause of lower respiratory tract infection and hospital visits during infancy and childhood. There is no vaccine for RSV and treatment is limited to supportive care (with fluids and oxygen until the illness runs its course).

Pneumonia, a common disease caused by a great diversity of infectious agents, is responsible for enormous morbidity and mortality worldwide. Pneumonia is the third leading cause of death worldwide and the leading cause of death due to infectious disease in industrialized countries. Bacteria are the most common cause of pneumonia in adults. Most community-acquired pneumonias are due to infections with *S. pneumoniae*, *Haemophilus influenzae*, and *Mycoplasma pneumoniae.* Lancet., 362:1991-200 (2003); Curr Opin Pulm Med., 6:226-233 (2000). The majority of late onset ventilator-associated pneumonias are caused by *S*. *aureus*, including antibiotic-resistant subtypes, *Pseudomonas* spp., *Klebsiella* spp., as well as *Acitenobacter* spp. Crit Care., 9:459-464 (2005).

Current therapies for respiratory tract infections involve the administration of anti-viral agents, anti-bacterial agents, or anti-fungal agents for the treatment, prevention, or amelioration of viral, bacterial, and fungal respiratory tract infections, respectively. Unfortunately, in some cases, there are no therapies available, or infections have been proven to be refractory to therapies, or the occurrence of side effects outweighs the benefits of the administration of a therapeutic agent. The use of anti-bacterial agents for treatment of bacterial respiratory tract infections may also produce side effects or result in resistant bacterial strains. The administration of anti-fungal agents may cause renal failure or bone marrow dysfunction and may not be effective against fungal infection in patients with suppressed immune systems. Additionally, the infection-causing microorganism (e.g., a virus, a bacterium, or a fungus) may be resistant or develop resistance to the administered therapeutic agent or combination of therapeutic agents. In fact, microorganisms that develop resistance to administered therapeutic agents often develop pleiotropic drug or multidrug resistance, that is, resistance to therapeutic agents that act by mechanisms different from the mechanisms of how the administered agents act. Thus, as a result of drug resistance, many infections prove refractory to a wide array of standard treatment protocols. Therefore, new therapies for the treatment, prevention, management, and/or amelioration of respiratory tract infections and symptoms thereof are needed.

U.S. Application Publication No. 2007/0053844 describes conductive formulations comprising a calcium salt in saline solution (e.g., 1.29% CaCl₂ dissolved in 0.9% NaCl). The formulations can alter the physical properties of the airway mucosal lining (such as the surface tension, surface elasticity, and bulk viscosity of the airway mucosal lining), and suppress exhaled particles. For example, formulations consisting of 1.29% CaCl₂ dissolved in 0.9% isotonic saline suppressed exhaled particles by more than 1000-fold.

### SUMMARY OF THE INVENTION

The present invention relates to pharmaceutical formulations as described in the appended claims.

In one aspect, the invention relates to pharmaceutical formulations useful for treating a respiratory tract infection or a pulmonary disease in an individual, comprising a calcium salt and a sodium salt, wherein the ratio of Ca⁺² to Na⁺ is from about 4:1 (mole:mole) to about 16:1 (mole:mole). Data provided herein show that when the ratio of Ca⁺² to Na⁺ is from about 4:1 (mole:mole) to about 16:1 (mole:mole), the combination of the two salts provide superior synergistic effects in reducing viral replication in a cell culture model of Influenza infection. Further, the formulations are highly effective in reducing viral replication of multiple strains of Influenza (e.g., Influenza A H1N1, Influenza A H3N2, Influenza B). Subsequent *in vivo* studies confirm that formulations with an 8:1 ratio of Ca⁺² to Na⁺ are also highly effective in treating influenza infection, as the formulations were shown to: (1) delay the onset and reduce the severity of fever, (2) prevent body weight loss, and (3) reduce nasal inflammatory cell counts in a ferret model of influenza. Similarly, in an *in vivo* mouse model of influenza, the formulations were shown to improve animal survival rate. In addition, the therapeutic activities of the pharmaceutical formulations as described herein is independent of pathogen, as the formulations were shown to be effective in (1) reducing human parainfluenza virus (hPIV) infectivity in a cell culture model, (2) reducing rhinovirus infectivity in a cell culture model, and (3) treating *Streptococcus pneumoniae* infection in a mouse model.

In certain embodiments, the ratio of Ca⁺² to Na⁺ is about 4:1 (mole:mole), about 4.5:1 (mole:mole), about 5:1 (mole:mole), about 5.5:1 (mole:mole), about 6:1 (mole:mole), about 6.5:1 (mole:mole), about 7:1 (mole:mole), about 7.5:1 (mole:mole), about 8:1 (mole:mole), about 8.5:1 (mole:mole), about 9:1 (mole:mole), about 9.5:1 (mole:mole), about 10:1 (mole:mole), about 10.5:1 (mole:mole), about 11:1 (mole:mole), about 11.5:1 (mole:mole), about 12:1 (mole:mole), about 12.5:1 (mole:mole), about 13:1 (mole:mole), about 13.5:1 (mole:mole), about 14:1 (mole:mole), about 14.5:1 (mole:mole), about 15:1 (mole:mole), about 15.5:1 (mole:mole), or about 16:1 (mole:mole).

In certain embodiments, the pharmaceutical formulation is a liquid formulation. In certain embodiments, the concentration of Ca²⁺ ion in the liquid formulation is from about 0.115 M to about 1.15 M, or from about 0.575 M to about 1.15 M.

In certain embodiments, the concentration of Na⁺ ion in the liquid formulation is from about 0.053 M to about 0.3 M, or from about 0.075 M to about 0.3 M.

The pharmaceutical formulations described herein may be hypotonic, isotonic or hypertonic as desired. For example, any of the pharmaceutical formulations described herein may have about 0.1X tonicity, about 0.25X tonicity, about 0.5X tonicity, about 1X tonicity, about 2X tonicity, about 3X tonicity, about 4X tonicity, about 5X tonicity, about 6X tonicity, about 7X tonicity, about 8X tonicity, about 9X tonicity, about 10X tonicity, at least about 1X tonicity, at least about 2X tonicity, at least about 3X tonicity, at least about 4X tonicity, at least about 5X tonicity, at least about 6X tonicity, at least about 7X tonicity, at least about 8X tonicity, at least about 9X tonicity, at least about 10X tonicity, between about 0.1X to about 1X, between about 0.1X to about 0.5X, between about 0.5X to about 2X, between about 1X to about 4X, between about 1X to about 2X, between about 2X to about 10X, or between about 4X to about 8X.

In certain embodiments, the pharmaceutical formulation comprises a calcium salt that is selected from the group consisting of calcium chloride, calcium carbonate, calcium acetate, calcium phosphate, calcium alginate, calcium stearate, calcium sorbate, calcium sulfate, calcium gluconate, calcium lactate and calcium citrate. In an exemplary embodiment, the calcium salt is calcium chloride or calcium lactate. In another exemplary embodiment, the calcium salt is calcium citrate, calcium lactate, or calcium sulfate.

In certain embodiments, the pharmaceutical formulation comprises a sodium salt that is selected from the group consisting of sodium chloride, sodium acetate, sodium bicarbonate, sodium carbonate, sodium sulfate, sodium stearate, sodium ascorbate, sodium benzoate, sodium biphosphate, sodium phosphate, sodium bisulfite, sodium citrate, sodium borate, sodium gluconate, sodium metasilicate, and sodium lactate. In an exemplary embodiment, the sodium salt is sodium chloride. In another exemplary embodiment, the sodium salt is sodium citrate, sodium lactate, or sodium sulfate.

In certain embodiments, the pharmaceutical formulation is a dry powder formulation. In certain embodiments, the calcium salt is present in the dry powder formulation in an amount of from about 19.5% to about 90% (w/w).

In an exemplary embodiment, the calcium salt of the dry powder formulation is calcium lactate, calcium citrate, calcium sulfate, calcium chloride, or a combination thereof.

In an exemplary embodiment, the sodium salt of the dry powder formulation is sodium lactate, sodium citrate, sodium sulfate, sodium chloride, or a combination thereof.

In certain embodiments, the pharmaceutical formulation is formulated to deliver a calcium dose of about 0.001 mg/kg body weight/dose to about 10 mg/kg body weight/dose to the lungs. In certain embodiments, the pharmaceutical formulation is formulated to provide a sodium dose of about 0.001 mg/kg body weight/dose to about 10 mg/kg body weight/dose to the lungs. In certain embodiments, the pharmaceutical formulation is formulated to deliver a calcium dose of about 0.001 mg/kg body weight/dose to about 10 mg/kg body weight/dose to the nasal cavity. In certain embodiments, the pharmaceutical formulation is formulated to provide a sodium dose of about 0.001 mg/kg body weight/dose to about 10 mg/kg body weight/dose to the nasal cavity.

In certain embodiments, the pharmaceutical formulation further comprises an additional therapeutic agent.

In certain embodiments, the pharmaceutical formulation further comprises an excipient. Exemplary excipients include lactose, glycine, alanine, leucine, isolucine, trehalose, dipalmitoylphosphosphatidylcholine (DPPC), diphosphatidyl glycerol (DPPG), 1,2-Dipalmitoyl-sn-glycero-3-phospho-L-serine (DPPS), 1,2-Dipalmitoyl-sn-glycero-3-phosphocholine (DSPC), 1,2-Distearoyl-sn-glycero-3-phosphoethanolamine (DSPE), 1-palmitoyl-2-oleoylphosphatidylcholine (POPC), polyoxyethylene-9-lauryl ether, sorbitan trioleate (Span 85), glycocholate, surfactin, tyloxapol, sodium phosphate, dextran, dextrin, mannitol, maltodextrin, human serum albumin, recombinant human serum albumin, or biodegradable polymers.

In certain embodiments, the pharmaceutical formulation is a unit dose formulation.

Also described herein is a method for treating (including prophylactically treating) a respiratory tract infection, comprising administering to an individual having a respiratory tract infection, exhibiting symptoms of a respiratory tract infection, or at risk of contracting a respiratory tract infection, an effective amount of a pharmaceutical formulation comprising a calcium salt and a sodium salt, wherein the ratio of Ca⁺² to Na⁺ is from about 4:1 (mole:mole) to about 16:1 (mole:mole).

Also described herein is a method for reducing the spread of a respiratory tract infection, comprising administering to an individual having a respiratory tract infection, exhibiting symptoms of a respiratory tract infection, or at risk of contracting a respiratory tract infection, an effective amount of a pharmaceutical formulation comprising a calcium salt and a sodium salt, wherein the ratio of Ca⁺² to Na⁺ is from about 4:1 (mole:mole) to about 16:1 (mole:mole).

In certain embodiments, the respiratory tract infection is a bacterial infection, such as bacterial pneumonia. In certain embodiments, the bacterial infection is caused by a bacterium selected from the group consisting of *Streptocuccus pneumoniae* (also referred to as pneumococcus), *Staphylococcus aureus*, *Streptococcus agal-actiac, Streptococcus pyogenes, Haemophilus influenzae*, *Haemophilus parainfluenzae, Klebsiella pneumoniae*, *Escherichia coli, Pseudomonas aeruginosa, Moraxella catarrhalis*, *Chlamydophila pneumoniae*, *Mycoplasma pneumoniae, Legionella pneumophila*, *Serratia marcescens*, *Burkholderia cepacia, Burkholderia pseudomallei*, *Bacillus anthracis*, *Bacillus cereus, Bordatella pertussis, Stenotrophomonas maltophilia*, a bacterium from the citrobacter family, a bacterium from the ecinetobacter family, and *Mycobacterium tuberculosis.*

In certain embodiments, the respiratory tract infection is a viral infection, such as influenza or viral pneumonia. In certain embodiments, the viral infection is caused by a virus selected from the group consisting of influenza virus (e.g., Influenza virus A, Influenza virus B), respiratory syncytial virus, adenovirus, metapneumovirus, cytomegalovirus, palainfluenza virus (e.g., hPIV-1, hPIV-2, hPIV-3, hPIV-4), rhinovirus, adenovirus, coxsackie virus, echo virus, corona virus, herpes simplex virus, SARS-coronavirus, and smallpox.

In certain embodiments, the respiratory tract infection is a fungal infection. In certain embodiments, the fungal infection is caused by a fungus selected from the group consisting of *Histoplasma capsulatum*, *Cryptococcus neoformans*, *Pneumocystis jiroveci*, *Coccidioides immitis*, *Candida albicans*, and *Pneumocystis jirovecii* (which causes pneumocystis pneumonia (PCP), also called pneumocystosis).

In certain embodiments, the respiratory tract infection is a parasitic infection. In certain embodiments, the parasitic infection is caused by a parasite selected from the group consisting of *Toxoplasma gondii* and *Strongyloides stercoralis.*

Also described herein is a method for treating (including prophylactically treating) an individual with a pulmonary disease (e.g., an individual having a pulmonary disease, exhibiting symptoms of a pulmonary disease, or susceptible to a pulmonary disease), comprising administering to the respiratory tract of the individual an effective amount of a pharmaceutical formulation comprising a calcium salt and a sodium salt, wherein the ratio of Ca⁺² to Na⁺ is from about 4:1 (mole:mole) to about 16:1 (mole:mole).

Also described herein is a method for treating (including prophylactically treating) an acute exacerbation of a chronic pulmonary disease in an individual, comprising administering to the respiratory tract of the individual in need thereof (e.g., an individual having an acute exacerbation of a pulmonary disease, exhibiting symptoms of an acute exacerbation of a pulmonary disease, or susceptible to an acute exacerbation of a pulmonary disease) an effective amount of a pharmaceutical formulation comprising a calcium salt and a sodium salt, wherein the ratio of Ca⁺² to Na⁺ is from about 4:1 (mole:mole) to about 16: (mole:mole). Exemplary pulmonary diseases include asthma (*e.g*., allergic/atopic, childhood, late-onset, cough-variant, or chronic obstructive), airway hyperresponsiveness, allergic rhinitis (seasonal or non-seasonal), bronchiectasis, chronic bronchitis, emphysema, chronic obstructive pulmonary disease, cystic fibrosis, early life wheezing, and the like.

The invention also relates to a pharmaceutical formulation as described herein for use in treating a respiratory tract infection, for reducing the spread of a respiratory tract infection, for treating a pulmonary disease, or for treating an acute exacerbation of a chronic pulmonary disease.

### BRIEF DESCRIPTION OF THE DRAWINGS

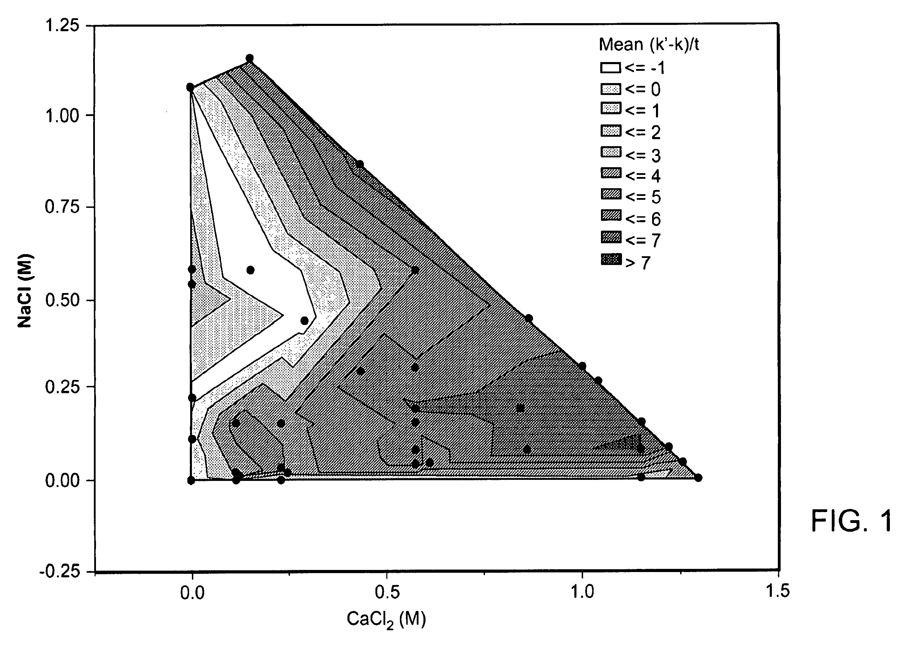

Figure 1 is a contour plot showing the effect of changing sodium and calcium concentrations on Influenza A/WSN/33/1 viral replication. To normalize the data from several experiments, the change of viral replication rate in each treated condition, as compared to the zero salt condition, was determined for each experiment. The X-axis depicts increasing CaCl₂ concentration and the Y-axis depicts increasing NaCl concentration. Each dot represents a formulation tested with at least three replicates per test. Reduced viral infectivity is shown by increasing darkness (*i.e*., higher numbers and darker shades represent formulations having greater effect on reducing Influenza viral replication).

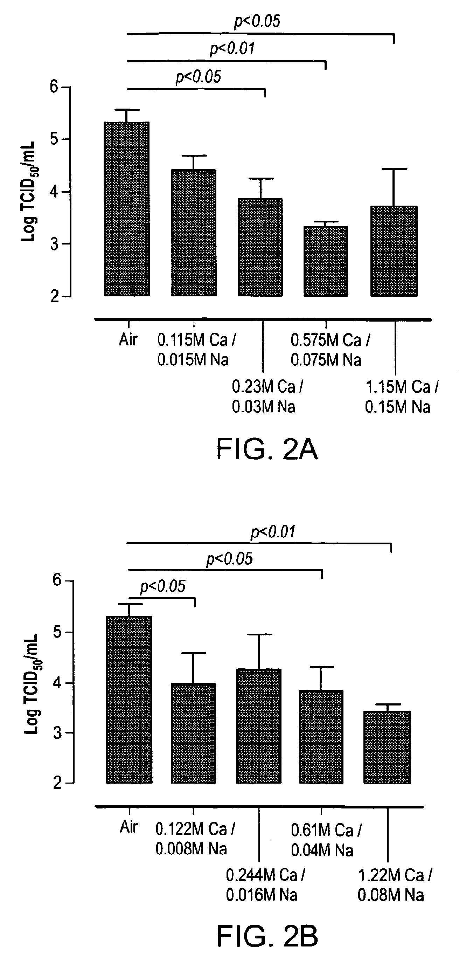

Figure 2A shows the dose responsive effects of liquid formulations with a Ca²⁺:Na⁺ ratio at 8:1 (mole:mole). Figure 2B shows the dose responsive effects of liquid formulations with a Ca²⁺:Na⁺ ratio at 16:1 (mole:mole). In both cases, cells were treated with the liquid formulations 1 hour before infection.

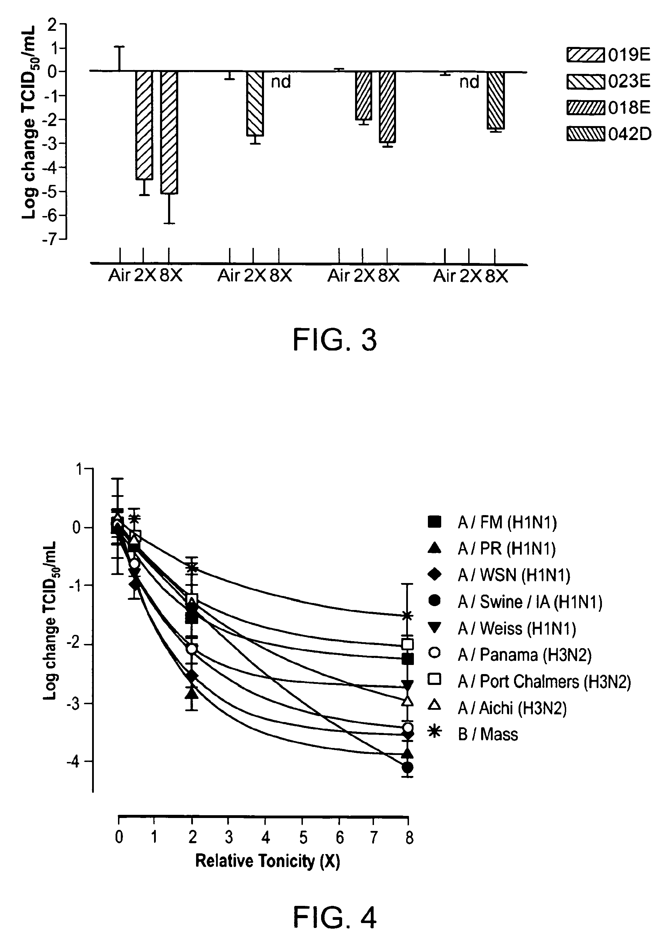

Figure 3 shows that the formulations reduced influenza infectivity in normal human bronchial epithelial (NHBE) cells. NHBE cells from four different donors were treated with the indicated formulations and infected with Influenza A/Panama/2007/99. Viral titers were determined in apical washes 24 hours after infection. Data were normalized to the untreated (air) control and presented as the log₁₀ change in TCID₅₀/mL from the control. Data are the mean ± SD for 2 to 3 replicates per condition. N.D. = not determined.

Figure 4 shows that the formulations reduced the infectivity of multiple strains of Influenza viruses in Calu-3 cells in a dose-responsive manner. Calu-3 cells were treated with formulations that were 0.5X, 2X and 8X in tonicity (1X = isotonic), with an 8:1 molar ratio of Ca²⁺:Na⁺. Viral titers were determined 24 hours after treatment and the fold reduction of viral titers relative to an untreated control was calculated for each viral strain. Influenza viral strains tested in this example are shown in the legend and in Table 2.

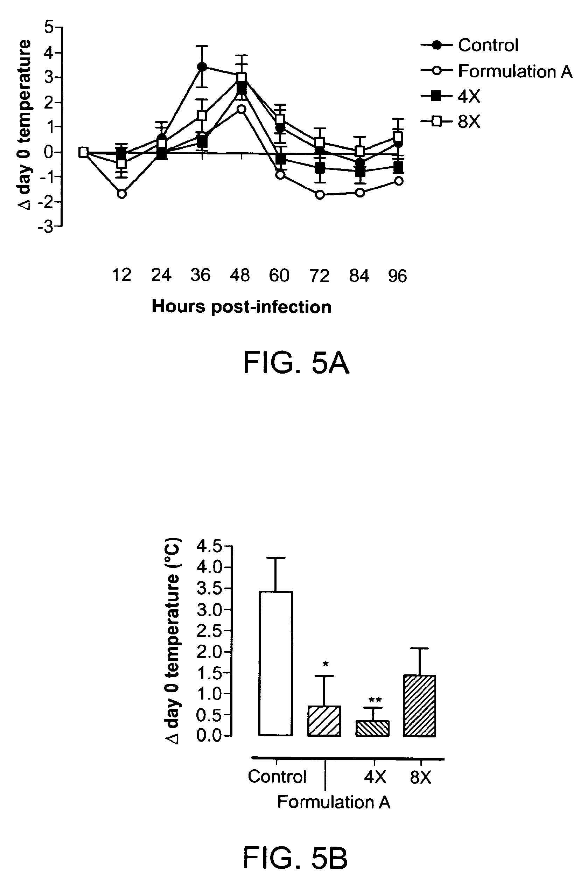

Figure 5 shows that treatment with calcium:sodium formulations delayed the onset of fever and reduced body temperatures in influenza infected ferrets. Body temperature changes (mean ± SEM) of control ferrets (closed circles), FORMULATION A treated ferrets (open circles), 4X treated ferrets (closed boxes), or 8X treated ferrets (open boxes) are depicted. Figure 5A shows that the treated ferrets exhibited delayed onset of fever and had lower body temperatures over the course of the study (*p*<0.0001 Two-way ANOVA). Figure 5B shows that the treated ferrets had lower body temperatures at the time of peak fever in the control animals (36 hours post-infection; **p<0.05, **p<0.01* Mann-Whitney U test; n=9 for control, n=9 for FORMULATION A treated, and n=10 for 4X and 8X treated).

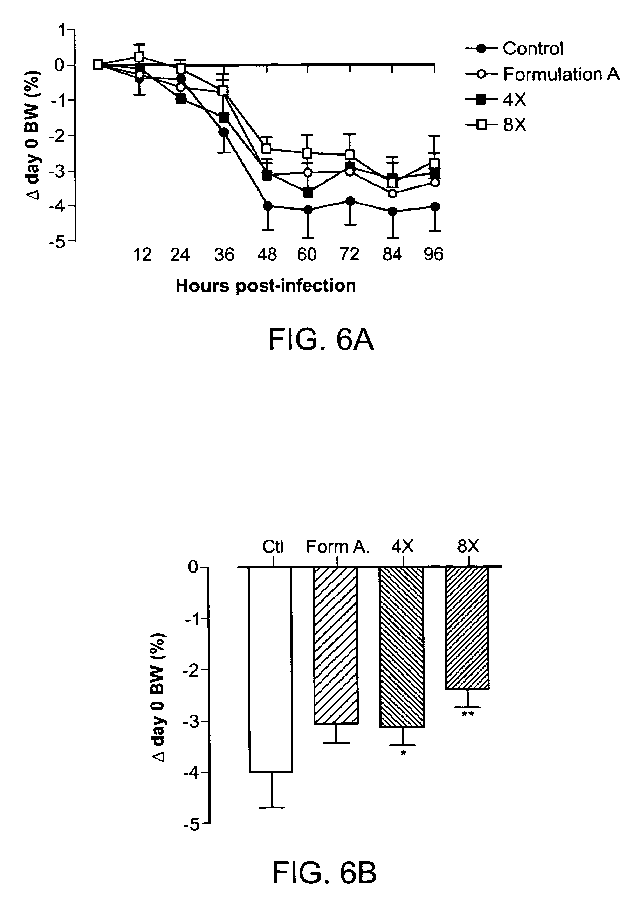

Figure 6 shows that treatment with calcium:sodium formulations prevented body weight loss in influenza infected ferrets. Percent body weight loss from time zero in control ferrets (closed circles), FORMULATION A treated ferrets (open circles), 4X treated ferrets (closed boxes), or 8X treated ferrets (open boxes) are depicted. Figure 6A shows that the treated ferrets exhibited less body weight loss over the course of the study (*p*<0.0001 Two-way ANOVA). Figure 6B shows that the treated ferrets had less body weight loss at the time of peak loss in the control animals (48 hours post-infection; **p<0.05, **p<0.01* Mann-Whitney U test; n=10 for all groups except FORMULATION A, n=9).

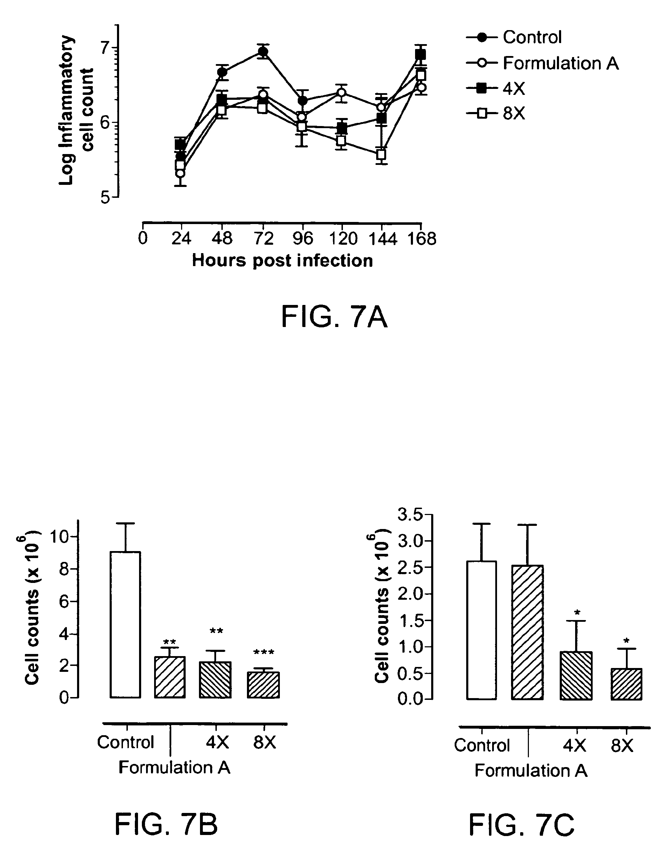

Figure 7 shows that treatment with calcium:sodium formulations dampened the inflammatory response to influenza infection in ferrets. Nasal washes were performed once daily at the indicated times and the number of inflammatory cells in each nasal wash were enumerated. Nasal wash samples that had noticeable amounts of blood were discarded for analysis at each timepoint. The mean (±SEM) control ferrets (closed circles), FORMULATION A treated ferrets (open circles), 4X treated ferrets (closed boxes), or 8X treated ferrets (open boxes) are depicted. Figure 7A shows that treatments with the calcium:sodium formulations reduced the number of inflammatory cells in nasal wash samples to statistically significant levels over time (*p*<0.0001 Two way ANOVA). Figure 7B shows the reduction at the peak of inflammatory cell infiltration in treated animals, as compared to the controls animals (72 hours; * **p*<*0.01* and ****P*<*0.001* Mann-Whitney U test; n=5 for control, n=6 for FORMULATION A and 4X treated, and n=7 for 8X treated at the 72 hours timepoint). Figure 7C shows the reduction of inflammatory cells in treated animals at 120 hours post-infection (**p*<*0.05* Mann-Whitney U test; n=6 for control, n=5 for FORMULATION A treated, n=6 for 4X treated, and n=7 for 8X treated).

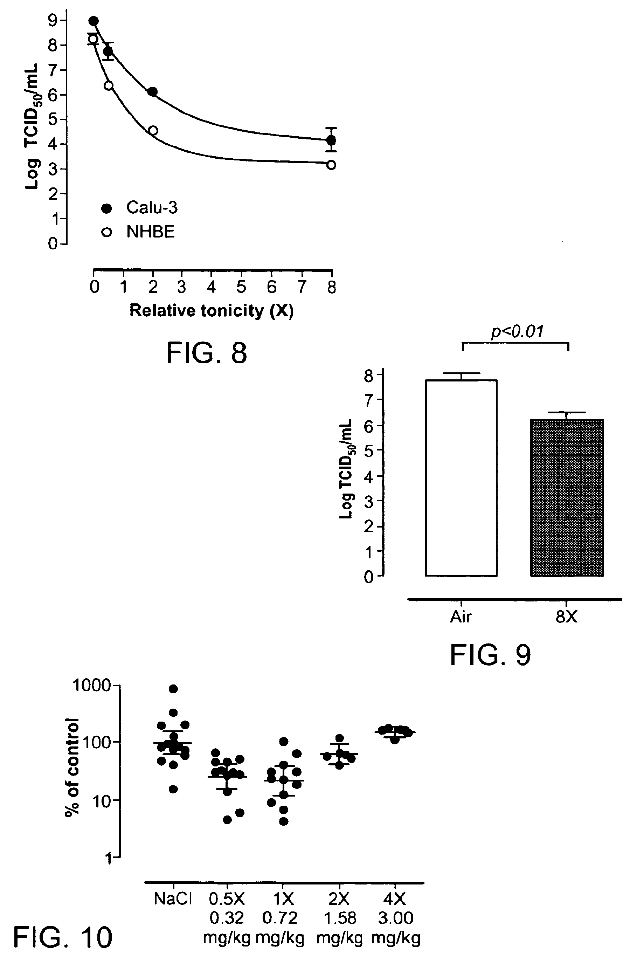

Figure 8 shows that calcium:sodium formulations reduced hPIV-3 infection in Calu-3 or Normal Human Bronchial Epithelial (NHBE) cells in a dose-responsive manner. Calu-3 (closed circles) or NHBE (open circles) cells were treated with calcium:sodium formulations (0.5X, 2X or 8X tonicity; 8:1 molar ratio of Ca²⁺:Na⁺) 1 hour before infection with human parainfluenza 3 (hPIV-3). Viral titer was determined in the apical washes of cells 24 hours after infection by TCID₅₀ assay using MK-2 cells. The titer of parainfluenza in the apical washes was reduced in a dose responsive manner. Data from each cell type were analyzed using a one-way ANOVA and Tukey's multiple comparison tests.

Figure 9 shows that calcium:sodium formulations reduced rhinorivus infection in Calu-3 cells. Calu-3 cells were treated with the calcium:sodium formulation (8X tonicity; 8:1 molar ratio of Ca²⁺:Na⁺). One hour later, cells were infected with rhinovirus and three hours later, cells were washed to remove unattached virus. Viral titer was determined from the apical surface of the cultures by TCID50 assay 24 hours after infection. Each data point is the mean + SD of duplicate wells and is representative of two independent experiments.

Figure 10 shows that calcium:sodium formulations (Ca²⁺:Na⁺ at 8:1 molar ratio) reduced lung bacterial burden in a dose responsive manner. Mice were treated with the indicated formulations using a PariLC Sprint nebulizer and subsequently infected with *S*. *pneumoniae.* The lung bacterial burden in each animal is shown. Each circle represents data from a single animal and the bar depicts the geometric mean with the 95% confidence interval. Data for the NaCl, 0.5X and 1X groups are pooled from two or three independent experiments. Data from the 2X and 4X groups are from a single experiment.

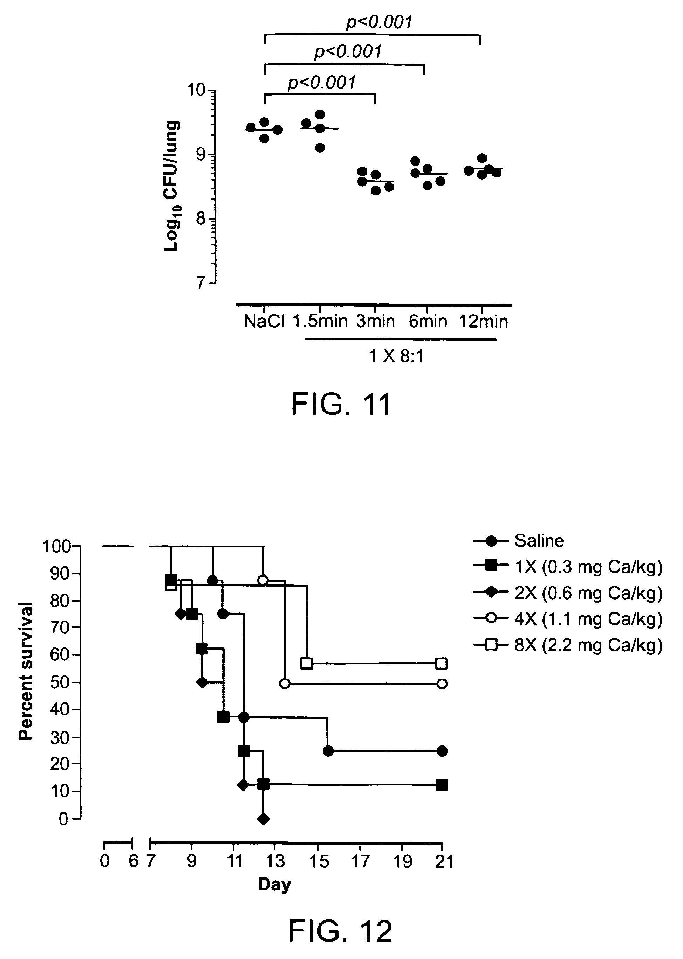

Figure 11 shows that increasing calcium dose with longer nebulization times does not significantly impact therapeutic activities. Mice were treated with Saline (NaCl) or a calcium:sodium formulation (1X tonicity = isotonic; Ca²⁺:Na⁺ at 8:1 molar ratio) using a Pari LC Sprint nebulizer and subsequently infected with *S*. *pneumoniae.* The lung bacterial burden in each animal is shown. Each circle represents data from a single animal and the bar depicts the geometric mean. Dosing times of 3 minutes or greater significantly reduced bacterial burdens relative to controls (one-way ANOVA; Tukey's multiple comparison post-test).

Figure 12 shows that the calcium:sodium formulations were effective in treating Influenza viral infection in a mouse influenza model. BALB/c mice (n=7-8 per group) were treated with each of the indicated formulations 3 hours before infection with Influenza A/PR/8 (H1N1). Mice were subsequently treated 3 hours after infection and then BID for a total of 11 days. Animal survival, changes in animal body temperature and changes in body weight were tracked for 21 days. Animals who exhibited body temperatures below 95°F were euthanized.

### DETAILED DESCRIPTION OF THE INVENTION

### 1. Overview

As described herein, *in vivo* and *in vitro* studies of the therapeutic activities of inhaled salt formulations that contain a calcium salt and a sodium salt were conducted. In the course of these studies, it was discovered that the relative ratio of calcium to sodium in the salt formulations affect the therapeutic activities of the formulations. The results of the *in vitro* and *in vivo* studies revealed that a narrow range of calcium to sodium ratios provided superior therapeutic activities. In particular, it was discovered that a ratio of calcium to sodium from about 4:1 to about 16:1 (mole:mole) provided superior therapeutic activities.

As described herein, the therapeutic activities of formulations having calcium:sodium ratios from about 4:1 to about 16:1 (mole:mole) have been demonstrated in several preclinical models. For example, the formulations were shown to, *inter alia*, have superior effects on reducing viral replication in cell culture model of influenza, reduce viral replications of multiple strains of Influenza (e.g., Influenza A H1N1, Influenza A H3N2, Influenza B), reduce the severity of fever and nasal inflammatory cell counts in an *in vivo* ferret influenza model, improve animal survival rate in an *in vivo* mouse influenza model, reduce viral replication of human parainfluenza virus (hPIV) in a cell culture model, reduce viral replication of rhinovirus in a cell culture model, and reduce lung bacterial burden of *Streptococcus pneumoniae* in a mouse pneumonia model. The data obtained from these studies demonstrate that the formulations are useful in treating (including prophylactically treating) respiratory tract infections caused by a broad spectrum of pathogens (e.g., multiple strains of influenza viruses, human parainfluenza virus, *S*. *pneumoniae*). The formulations are also useful for reducing the spread of a respiratory tract infection, treating a pulmonary disease, or treating an acute exacerbation of a chronic pulmonary disease.

### 2. Definitions

As used herein, the phrase "aerodynamically light particles" refers to particles having a tap density less than about 0.4 g/cm³. The tap density of particles of a dry powder may be obtained by the standard USP tap density measurement. Tap density is a common measure of the envelope mass density. The envelope mass density of an isotropic particle is defined as the mass of the particle divided by the minimum sphere envelope volume in which it can be enclosed. Features contributing to low tap density include irregular surface texture and porous structure.

The term "aerosol" as used herein refers to any preparation of a fine mist of particles (including liquid and non-liquid particles, *e.g*., dry powders, typically with a volume median geometric diameter of about 0.1 to about 30 microns or a mass median aerodynamic diameter of between about 0.5 and about 10 microns. Preferably the volume median geometric diameter for the aerosol particles is less than about 10 microns. The preferred volume median geometric diameter for aerosol particles is about 5 microns. For example, the aerosol can contain particles that have a volume median geometric diameter between about 0.1 and about 30 microns, between about 0.5 and about 20 microns, between about 0.5 and about 10 microns, between about 1.0 and about 3.0 microns, between about 1.0 and 5.0 microns, between about 1.0 and 10.0 microns, between about 5.0 and 15.0 microns. Preferably the mass median aerodynamic diameter is between about 0.5 and about 10 microns, between about 1.0 and about 3.0 microns, or between about 1.0 and 5.0 microns.

The term "pneumonia" is a term of art that refers to an inflammatory illness of the lung. Pneumonia can result from a variety of causes, including infection with bacteria, viruses, fungi, or parasites, and chemical or physical injury to the lungs. Typical symptoms associated with pneumonia include cough, chest pain, fever and difficulty breathing. Clinical diagnosis of pneumonia is well-known in the art and may include x-ray and/or examination of sputum.

The term "bacterial pneumonia" refers to pneumonia caused by bacterial infection, including for example, infection of the respiratory tract by *Streptococcus pneumoniae, Staphylococcus aureus, Streptococcus agalactiae, Haemophilus influenzae, Klebsiella pneumoniae, Escherichia coli, Pseudomonas aeruginosa, Moraxella catarrhalis, Chlamydophila pneumoniae, Mycoplasma pneumoniae* or *Legionella pneumophila.*

The term "viral pneumonia" refers to pneumonia caused by a viral infection. Viruses that commonly cause viral pneumonia include, for example, influenza virus, respiratory syncytial virus (RSV), adenovirus, and metapneumovirus. Herpes simplex virus is a rare cause of pneumonia for the general population, but is more common in newborns. People with weakened immune systems are also at risk for pneumonia caused by cytomegalovirus (CMV).

The term "respiratory tract" as used herein includes the upper respiratory tract (*e.g*., nasal passages, nasal cavity, throat, pharynx), respiratory airways (*e.g*., larynx, tranchea, bronchi, bronchioles) and lungs (*e.g*., respiratory bronchioles, alveolar ducts, alveolar sacs, alveoli).

The term "respiratory tract infection" is a term of art that refers to upper respiratory tract infections (*e.g*., infections of the nasal cavity, pharynx, larynx) and lower respiratory tract infections (*e.g*., infections of the trachea, primary bronchi, lungs) and combinations thereof. Typical symptoms associated with respiratory tract infections include nasal congestion, cough, running nose, sore throat, fever, facial pressure, sneezing, chest pain and difficulty breathing.

As used herein, "1X" tonicity refers to a solution that is isotonic relative to normal human blood and cells. Solutions that are hypotonic or hypertonic in comparison to normal human blood and cells are described relative to a 1X solution using an appropriate multiplier. For example, a hypotonic solution may have 0.1X, 0.25X or 0.5X tonicity, and a hypertonic solution may have 2X, 3X, 4X, 5X, 6X, 7X, 8X, 9X or 10X tonicity.

The term "dry powder" as used herein refers to a composition contains finely dispersed respirable dry particles that are capable of being dispersed in an inhalation device and subsequently inhaled by a subject. Such dry powder or dry particle may contain up to about 15% water or other solvent, or be substantially free of water or other solvent, or be anhydrous.

### 3. Pharmaceutical Formulations

In one aspect, the invention relates to pharmaceutical formulations that comprise a calcium salt and a sodium salt, wherein the ratio of Ca⁺² to Na⁺ is from about 4:1 (mole:mole) to about 16:1 (mole:mole). The pharmaceutical formulations are suitable for inhalation, and may be used to treat a respiratory tract infection, reduce the spread of a respiratory tract infection, treat a pulmonary disease, or treat an acute exacerbation of a chronic pulmonary disease in an individual in need thereof. Without wishing to be bound by any particular theory, it is believed that the therapeutic benefits provided by the salt formulations described herein, result from an increase in the amount of cation (Ca²⁺, Na⁺, or Ca²⁺ and Na⁺) in the respiratory tract (*e.g*., lung mucus or airway lining fluid) after administration of the salt formulation.

The pharmaceutical formulations can contain a ratio of Ca⁺² to Na⁺ from about 4:1 (mole:mole) to about 16:1 (mole:mole). For example, the formulations can contain a ratio of Ca⁺² to Na⁺ from about 5:1 (mole:mole) to about 16:1 (mole:mole), from about 6:1 (mole:mole) to about 16:1 (mole:mole), from about 7:1 (mole:mole) to about 16:1 (mole:mole), from about 8:1 (mole:mole) to about 16:1 (mole:mole), from about 9:1 (mole:mole) to about 16:1 (mole:mole), from about 10:1 (mole:mole) to about 16:1 (mole:mole), from about 11:1 (mole:mole) to about 16:1 (mole:mole), from about 12:1 (mole:mole) to about 16:1 (mole:mole), from about 13:1 (mole:mole) to about 16:1 (mole:mole), from about 14:1 (mole:mole) to about 16:1 (mole:mole), from about 15:1 (mole:mole) to about 16:1 (mole:mole).

Alternatively or in addition, the formulations can contain a ratio of Ca⁺² to Na⁺ from about 4:1 (mole:mole) to about 5:1 (mole:mole), from about 4:1 (mole:mole) to about 6:1 (mole:mole), from about 4:1 (mole:mole) to about 7:1 (mole:mole), from about 4:1 (mole:mole) to about 8:1 (mole:mole), from about 4:1 (mole:mole) to about 9:1 (mole:mole), from about 4:1 (mole:mole) to about 10:1 (mole:mole), from about 4:1 (mole:mole) to about 11:1 (mole:mole), from about 4:1 (mole:mole) to about 12:1 (mole:mole), from about 4:1 (mole:mole) to about 13:1 (mole:mole), from about 4:1 (mole:mole) to about 14:1 (mole:mole), from about 4:1 (mole:mole) to about 15:1 (mole:mole).

Alternatively or in addition, the formulations can contain a ratio of Ca⁺² to Na⁺ from about 4:1 (mole:mole) to about 12:1 (mole:mole), from about 5:1 (mole:mole) to about 11:1 (mole:mole), from about 6:1 (mole:mole) to about 10:1 (mole:mole), from about 7:1 (mole:mole) to about 9:1 (mole:mole).

Alternatively or in addition, the ratio of Ca⁺² to Na⁺ is about 4: (mole:mole), about 4.5:1 (mole:mole), about 5:1 (mole:mole), about 5.5:1 (mole:mole), about 6:1 (mole:mole), about 6.5:1 (mole:mole), 7:1 (mole:mole), about 7.5:1 (mole:mole), about 8:1 (mole:mole), about 8.5:1 (mole:mole), about 9:1 (mole:mole), about 9.5:1 (mole:mole), about 10:1 (mole:mole), about 10.5:1 (mole:mole), about 11:1 (mole:mole), about 11.5:1 (mole:mole), about 12:1 (mole:mole), about 12.5:1 (mole:mole), about 13:1 (mole:mole), about 13.5:1 (mole:mole), about 14:1 (mole:mole), about 14.5:1 (mole:mole), about 15:1 (mole:mole), about 15.5:1 (mole:mole), or about 16:1 (mole:mole).

In exemplary embodiments, the ratio of Ca⁺² to Na⁺ is about 8:1 (mole:mole) or about 16:1 (mole:mole).

Suitable calcium salts include, for example, calcium chloride, calcium carbonate, calcium acetate, calcium phosphate, calcium alginate, calcium stearate, calcium sorbate, calcium sulfate, calcium gluconate, calcium citrate, calcium lactate, and the like, or a combination thereof.

Certain calcium salts provide two or more moles of Ca²⁺ per mole of calcium salt upon dissolution. Such calcium salts may be particularly suitable to produce liquid or dry powder formulations that are dense in calcium, and therefore, can deliver an effective amount of cation (e.g., Ga²⁺, Na⁺, or Ca²⁺ and Na⁺). For example, one mole of calcium citrate provides three moles of Ca²⁺ upon dissolution. It is also generally preferred that the calcium salt is a salt with a low molecular weight and/or contain low molecular weight anions. Low molecular weight calcium salts, such as calcium salts that contain calcium ions and low molecular weight anions, are calcium dense relative to high molecular salts and calcium salts that contain high molecular weight anions. It is generally preferred that the calcium salt has a molecular weight of less than about 1000 g/mol, less than about 950 g/mol, less than about 900 g/mol, less than about 850 g/mol, less than about 800 g/mol, less than about 750 g/mol, less than about 700 g/mol, less than about 650 g/mol, less than about 600 g/mol, less than about 550 g/mol, less than about 510 g/mol, less than about 500 g/mol, less than about 450 g/mol, less than about 400 g/mol, less than about 350 g/mol, less than about 300 g/mol, less than about 250 g/mol, less than about 200 g/mol, less than about 150 g/mol, less than about 125 g/mol, or less than about 100 g/mol. In addition or alternatively, it is generally preferred that the calcium ion contributes a substantial portion of the weight to the overall weight of the calcium salt. It is generally preferred that the calcium ion weigh at least 10% of the overall calcium salt, at least 16%, at least 20%, at least 24.5%, at least 26%, at least 31%, at least 35%, or at least 38% of the overall calcium salt.

Alternatively or in addition, the pharmaceutical formulations include a suitable calcium salt that provides calcium, wherein the weight ratio of calcium to the overall weight of said calcium salt is between about 0.1 to about 0.5. For example, the weight ratio of calcium to the overall weight of said calcium salt is between about 0.15 to about 0.5, between about 0.18 to about 0.5, between about 0.2 to about 5, between about 0.25 to about 0.5, between about 0.27 to about 0.5, between about 0.3 to about 5, between about 0.35 to about 0.5, between about 0.37 to about 0.5, or between about 0.4 to about 0.5.

Suitable sodium salts include, for example, sodium chloride, sodium acetate, sodium bicarbonate, sodium carbonate, sodium sulfate, sodium stearate, sodium ascorbate, sodium benzoate, sodium biphosphate, sodium phosphate, sodium bisulfite, sodium citrate, sodium lactate, sodium borate, sodium gluconate, sodium metasilicate, and the like, or a combination thereof.

In addition to a calcium salt and a sodium salt, the pharmaceutical formulations of the invention can include any non-toxic salt form of the elements sodium, potassium, magnesium, calcium, aluminum, silicon, scandium, titanium, vanadium, chromium, cobalt, nickel, copper, manganese, zinc, tin, silver and similar elements. Suitable magnesium salts include, for example, magnesium carbonate, magnesium sulfate, magnesium stearate, magnesium trisilicate, magnesium chloride, and the like. Suitable potassium salts include, for example, potassium bicarbonate, potassium chloride, potassium citrate, potassium borate, potassium bisulfite, potassium biphosphate, potassium alginate, potassium benzoate, and the like. Additional suitable salts include cupric sulfate, chromium chloride, stannous chloride, and similar salts. Other suitable salts include zinc chloride, aluminum chloride and silver chloride.

The pharmaceutical formulations can be in any desired form, such as a liquid solution, emulsion, suspension, or a dry powder.

The pharmaceutical formulations are generally prepared in or comprise a physiologically acceptable carrier or excipient. For formulations in the form of liquid solutions, suspensions or emulsions, suitable carriers include, for example, aqueous, alcoholic/aqueous, and alcohol solutions, emulsions or suspensions, including solutions, emulsions or suspensions that contain water, saline, ethanol/water solutions, ethanol solutions, buffered media, propellants and the like. For formulations in the form of dry powders, suitable carrier or excipients include, for example, sugars (*e.g*., lactose, trehalose), sugar alcohols (e.g., mannitol, xylitol, sorbitol), amino acids (*e.g*., glycine, alanine, leucine, isoleucine), dipalmitoylphosphosphatidylcholine (DPPC), diphosphatidyl glycerol (DPPG), 1,2-Dipalmitoyl-sn-glycero-3-phospho-L-serine (DPPS), 1,2-Dipalmitoyl-sn-glycero-3-phosphocholine (DSPC), 1,2-Distearoyl-sn-glycero-3-phosphoethanolamine (DSPE), 1-palmitoyl-2-oleoylphosphatidylcholine (POPC), fatty alcohols, polyoxyethylene-9-lauryl ether, surface active fatty, acids, sorbitan trioleate (Span 85), glycocholate, surfactin, poloxomers, sorbitan fatty acid esters, tyloxapol, phospholipids, alkylated sugars, sodium phosphate, maltodextrin, human serum albumin (*e.g*., recombinant human serum albumin), biodegradable polymers (*e.g*., PLGA), dextran, dextrin, and the like. If desired, the salt formulations can also contain additives, preservatives, or fluid, nutrient or electrolyte replenishers (See, generally, Remington's Pharmaceutical Sciences, 17th Edition, Mack Publishing Co., PA, 1985).

The pharmaceutical formulations are preferably formulated for administration to the respiratory tract, for example as an aerosol.

The pharmaceutical formulations can comprise multiple doses or be a unit dose composition as desired.

The pharmaceutical formulations preferably contain concentrations of calcium and sodium that permit convenient administration of an effective amount of the formulation to the respiratory tract. For example, it is generally desirable that liquid formulations not be so dilute so as to require a large amount of the formulation to be nebulized in order to deliver an effective amount to the respiratory tract of a subject. Long administration periods are disfavored, and generally the formulation should be concentrated enough to permit an effective amount to be administered to the respiratory tract (*e.g*., by inhalation of aerosolized formulation, such as nebulized liquid or aerosolized dry powder) or nasal cavity in no more than about 120 minutes, no more than about 90 minutes, no more than about 60 minutes, no more than about 45 minutes, no more than about 30 minutes, no more than about 25 minutes, no more than about 20 minutes, no more than about 15 minutes, no more than about 10 minutes, no more than about 7.5 minutes, no more than about 5 minutes, no more than about 4 minutes, no more than about 3 minutes, no more than about 2 minutes, no more than about 1 minute, no more than about 45 seconds, or no more than about 30 seconds.

For example, a liquid pharmaceutical formulation may contain from about 0.115 M to 1.15 M Ca²⁺ ion, from about 0.116 M to 1.15 M Ca²⁺ ion, from about 0.23 M to 1.15 M Ca²⁺ ion, from about 0.345 M to 1.15 M Ca²⁺ ion, from about 0.424 M to 1.15 M Ca²⁺ ion, from about 0.46 M to 1.15 M Ca²⁺ ion, from about 0.575 M to 1.15 M Ca²⁺ ion, from about 0.69 M to 1.15 M Ca²⁺ ion, from about 0.805 M to 1.15 M Ca²⁺ ion, from about 0.849 M to 1.15 M Ca²⁺ ion, or from about 1.035 M to 1.15 M Ca²⁺ ion. The solubility of certain calcium salts (e.g., calcium carbonate, calcium citrate) can limit the preparation of solutions. In such situations, the liquid formulation may be in the form of a suspension that contains the equivalent amount of calcium salt that would be needed to achieve the desired molar concentration.

The concentration of Na⁺ ion in a liquid pharmaceutical formulation will depend on the concentration of Ca²⁺ ion in the liquid formulation, and the desired Ca²⁺: Na⁺ ratio. For example, the liquid formulation may contain from about 0.053 M to 0.3 M Na⁺ ion, from about 0.075 M to 0.3 M Na⁺ ion, from about 0.106 M to 0.3 M Na⁺ ion, from about 0.15 M to 0.3 M Na⁺ ion, from about 0.225 M to 0.3 M Na⁺ ion, from about 0.008 M to 0.3 M Na⁺ ion, from about 0.015 M to 0.3 M Na⁺ ion, from about 0.016 M to 0.3 M Na⁺ ion, from about 0.03 M to 0.3 M Na⁺ ion, from about 0.04 M to 0.3 M Na⁺ ion, from about 0.08 M to 0.3 M Na⁺ ion, from about 0.01875 M to 0.3 M Na⁺ ion, from about 0.0375 M to 0.3 M Na⁺ ion, from about 0.075 M to 0.6 M Na⁺ ion, from about 0.015 M to 0.6 M Na⁺ ion, or from about 0.3 M to 0.6 M Na⁺ ion.

The pharmaceutical formulations described herein may be hypotonic, isotonic or hypertonic as desired. Aqueous liquid formulations may vary in tonicity and in the concentrations of calcium salt and sodium salt that are present in the formulations.

The pharmaceutical formulations described herein may be hypotonic, isotonic or hypertonic as desired. For example, any of the pharmaceutical formulations described herein may have about 0.1X tonicity, about 0.25X tonicity, about 0.5X tonicity, about 1X tonicity, about 2X tonicity, about 3X tonicity, about 4X tonicity, about 5X tonicity, about 6X tonicity, about 7X tonicity, about 8X tonicity, about 9X tonicity, about 10X tonicity, at least about 1X tonicity, at least about 2X tonicity, at least about 3X tonicity, at least about 4X tonicity, at least about 5X tonicity, at least about 6X tonicity, at least about 7X tonicity, at least about 8X tonicity, at least about 9X tonicity, at least about 10X tonicity, between about 0.1X to about 1X, between about 0.1X to about 0.5X, between about 0.5X to about 2X, between about 1X to about 4X, between about 1X to about 2X, between about 2X to about 10X, or between about 4X to about 8X. For example, exemplary pharmaceutical formulations may contain about 0.053M CaCl₂ and about 0.0066M NaCl (0.5X tonicity, at 8:1 Ca²⁺:Na⁺ ratio), about 0.106M CaCl₂ and about 0.013M NaCl (1X tonicity (isotonic), at 8:1 Ca²⁺:Na⁺ ratio), about 0.212M CaCl₂ and about 0.0265M NaCl (2X tonicity, at 8:1 Ca²⁺:Na⁺ ratio), about 0.424M CaCl₂ and about 0.053M NaCl (4X tonicity, at 8:1 Ca²⁺:Na⁺ ratio), or about 0.849 M CaCl₂ and about 0.106 M NaCl (8X tonicity, at 8:1 Ca²⁺:Na⁺ ratio).

Preferred calcium salts for liquid formulations include, e.g., calcium chloride, calcium lactate, calcium citrate, or calcium sulfate. Preferred sodium salts for liquid formulations include, e.g., sodium chloride, sodium lactate, sodium citrate, or sodium sulfate.

Dry powder pharmaceutical formulations generally contain at least about 5% calcium salt by weight, 10% calcium salt by weight, about 15% calcium salt by weight, at least about 19.5% calcium salt by weight, at least about 20% calcium salt by weight, at least about 22% calcium salt by weight, at least about 25.5% calcium salt by weight, at least about 30% calcium salt by weight, at least about 37% calcium salt by weight, at least about 40% calcium salt by weight, at least about 48.4% calcium salt by weight, at least about 50% calcium salt by weight, at least about 60% calcium salt by weight, at least about 70% calcium salt by weight, at least about 75% calcium salt by weight, at least about 80% calcium salt by weight, at least about 85% calcium salt by weight, at least about 90% calcium salt by weight, or at least about 95% calcium salt by weight.

Alternatively or in addition, dry powder formulations may contain a calcium salt which provides Ca⁺² in an amount of at least about 5% Ca⁺² by weight, at least about 7% Ca⁺² by weight, at least about 10% Ca⁺² by weight, at least about 11% Ca⁺² by weight, at least about 12% Ca⁺² by weight, at least about 13% Ca⁺² by weight, at least about 14% Ca⁺² by weight, at least about 15% Ca⁺² by weight, at least about 17% Ca⁺² by weight, at least about 20% Ca⁺² by weight, at least about 25% Ca⁺² by weight, at least about 30% Ca⁺² by weight, at least about 35% Ca⁺² by weight, at least about 40% Ca⁺² by weight, at least about 45% Ca⁺² by weight, at least about 50% Ca⁺² by weight, at least about 55% Ca⁺² by weight, at least about 60% Ca⁺² by weight, at least about 65% Ca⁺² by weight or at least about 70% Ca⁺² by weight.

The amount of sodium salt in the dry powder pharmaceutical formulation will depend on the amount of calcium salt in the formulation and the desired calcium:sodium ratio. For example, the dry powder formulation may contain at least about 1.6% sodium salt by weight, at least about 5% sodium salt by weight, at least about 10% sodium salt by weight, at least about 13% sodium salt by weight, at least about 15% sodium salt by weight, at least about 20% sodium salt by weight, at least about 24.4% sodium salt by weight, at least about 28% sodium salt by weight, at least about 30% sodium salt by weight, at least about 30.5% sodium salt by weight, at least about 35% sodium salt by weight, at least about 40% sodium salt by weight, at least about 45% sodium salt by weight, at least about 50% sodium salt by weight, at least about 55% sodium salt by weight, or at least about 60% sodium salt by weight.

Alternatively or in addition, dry powder pharmaceutical formulations may contain a sodium salt which provides Na⁺ in an amount of at least about 0.1% Na⁺ by weight, at least about 0.5% Na⁺ by weight, at least about 1% Na⁺ by weight, at least about 2% Na⁺ by weight, at least about 3% Na⁺ by weight, at least about 4% Na⁺ by weight, at least about 5% Na⁺ by weight, at least about 6% Na⁺ by weight, at least about 7% Na⁺ by weight, at least about 8% Na⁺ by weight, at least about 9% Na⁺ by weight, at least about 10% Na⁺ by weight, at least about 11% Na⁺ by weight, at least about 12% Na⁺ by weight, at least about 14% Na⁺ by weight, at least about 16% Na⁺ by weight, at least about 18% Na⁺ by weight, at least about 20% Na⁺ by weight, at least about 22% Na⁺ by weight, at least about 25% Na⁺ by weight, at least about 27% Na⁺ by weight, at least about 29% Na⁺ by weight, at least about 32% Na⁺ by weight, at least about 35% Na⁺ by weight, at least about 40% Na⁺ by weight, at least about 45% Na⁺ by weight, at least about 50% Na⁺ by weight, or at least about 55% Na⁺ by weight.

Preferred calcium salts for dry powder formulations include, e.g., calcium chloride, calcium lactate, calcium citrate, or calcium sulfate. Preferred sodium salts for dry powder formulations include, e.g., sodium chloride, sodium lactate, sodium citrate, or sodium sulfate.

Preferred excipients for dry powder pharmaceutical formulations (such as the hydrophobic amino acid leucine) can be present in the formulations in an amount of about 50% or less (w/w). For example, a dry powder formulation may contain the amino acid leucine in an amount of about 50% or less by weight, about 45% or less by weight, about 40% or less by weight, about 35% or less by weight, about 30% or less by weight, about 25% or less by weight, about 20% or less by weight, about 18% or less by weight, about 16% or less by weight, about 15% or less by weight, about 14% or less by weight, about 13% or less by weight, about 12% or less by weight, about 11% or less by weight, about 10% or less by weight, about 9% or less by weight, about 8% or less by weight, about 7% or less by weight, about 6% or less by weight, about 5% or less by weight, about 4% or less by weight, about 3% or less by weight, about 2% or less by weight, or about 1% or less by weight. Exemplary excipients may include leucine, maltodextrin, mannitol, any combination of leucine, maltodextrin, and mannitol, or any other excipients disclosed herein or commonly used in the art.

Dry powder formulations are prepared with the appropriate particle diameter, surface roughness, and tap density for localized delivery to selected regions of the respiratory tract. For example, higher density or larger particles may be used for upper airway delivery. Similarly, a mixture of different sized particles can be administered to target different regions of the lung in one administration. For example, it has been found that dry powder comprising aerodynamically light particles are suitable for inhalation into the lungs.

Dry powder formulations ("DPFs") with large particle size have improved flowability characteristics, such as less aggregation (Visser, J., Powder Technology 58: 1-10 (1989)), easier aerosolization, and potentially less phagocytosis. Rudt, S. and R. H. Muller. J. Controlled Release, 22: 263-272 (1992); Tabata Y., and Y. Ikada. J. Biomed. Mater. Res. 22: 837-858 (1988). Dry powder aerosols for inhalation therapy are generally produced with mean diameters primarily in the range of less than 5 microns, although dry powders that have any desired range in aerodynamic diameter can be produced. Ganderton D., J. Biopharmaceutical Sciences, 3:101-105 (1992); Gonda, I. "Plysico-Chemical Principles in Aerosol Delivery." in Topics in Pharmaceutical Sciences 1991, Crommelin, D. J. and K. K. Midha, Eds., Medpharm Scientific Publishers, Stuttgart, pp. 95-115 (1992). Large "carrier" particles (containing no pharmaceutical formulation) can be co-delivered with therapeutic aerosols to aid in achieving efficient aerosolization among other possible benefits. French, D. L., Edwards, D. A. and Niven, R. W., J. Aerosol Sci. 27: 769-783 (1996). Particles with degradation and release times ranging from seconds to months can be designed and fabricated by established methods in the art.

Generally, pharmaceutical formulations that are dry powders may be produced by spray drying, freeze drying, jet milling, single and double emulsion solvent evaporation, and super-critical fluids. Preferably, dry powder formulations are produced by spray drying, which entails preparing a solution containing the salt and other components of the formulation, spraying the solution into a closed chamber, and removing the solvent with a heated gas steam.

Spray dried powders that contain calcium and sodium salts with sufficient solubility in water or aqueous solvents, such as calcium chloride, calcium lactate, and sodium chloride, sodium citrate can be readily prepared using conventional methods. Some salts, such as calcium citrate, and calcium sulfate, have low solubility in water and other aqueous solvents. Spray dried powders that contain such salts can be prepared using any suitable method. One suitable method involves combining other more soluble salts in solution and permitting reaction (precipitation reaction) to produce the desired salt for the dry powder formulation. For example, if a dry powder formulation comprising calcium citrate and sodium chloride is desired, a solution containing the high solubility salts calcium chloride and sodium citrate can be prepared. The precipitation reaction leading to calcium citrate is 3 CaCl₂ + 2 Na₃Cit → Ca₃Cit₂ + 6 NaCl. Additionally, for example, if a dry powder formulation comprising calcium sulfate and sodium chloride is desired, a solution containing the high solubility salts calcium chloride and sodium sulfate can be prepared. The precipitation reaction leading to calcium sulfate is CaCl₂ + Na2SO₄ → CaSO₄ + 2 NaCl. It is preferable that the sodium salt is fully dissolved before the calcium salt is added and that the solution is continuously stirred. The precipitation reaction can be allowed to go to completion or stopped before completion, *e.g*., by spray drying the solution, as desired.

Alternatively, two saturated or sub-saturated solutions are fed into a static mixer in order to obtain a saturated or supersaturated solution post-static mixing. Preferably, the post-spray drying solution is supersaturated. The two solutions may be aqueous or organic, but are preferably substantially aqueous. The post-static mixing solution is then fed into the atomizing unit of a spray dryer. In a preferable embodiment, the post-static mixing solution is immediately fed into the atomizer unit. Some examples of an atomizer unit include a two-fluid nozzle, a rotary atomizer, or a pressure nozzle. Preferably, the atomizer unit is a two-fluid nozzle. In one embodiment, the two-fluid nozzle is an internally mixing nozzle, meaning that the gas impinges on the liquid feed before exiting to the most outward orifice. In another embodiment, the two-fluid nozzle is an externally mixing nozzle, meaning that the gas impinges on the liquid feed after exiting the most outward orifice.

The resulting solution may appear clear with fully dissolved salts or a precipitate may form. Depending on reaction conditions, a precipitate may form quickly or over time. Solutions that contain a light precipitate that results in formation of a stable homogenous suspension can be spray dried.

Dry powder formulations can also be prepared by blending individual components into the final pharmaceutical formulation. For example, a first dry powder that contains a calcium salt can be blended with a second dry powder that contains a sodium salt to produce a pharmaceutical formulation that contains a calcium salt and a sodium salt. If desired, additional dry powders that contain excipients (*e.g*., lactose) and/or other active ingredients (*e.g*., antibiotic, antiviral) can be included in the blend. The blend can contain any desired relative amounts or ratios of calcium salt, sodium salt, excipients and other ingredients (*e.g*., antibiotics, antivirals).

If desired, dry powders can be prepared using polymers that are tailored to optimize particle characteristics including: i) interactions between the agent (*e.g*., salt, other active ingredient such as antibiotic or antiviral) to be delivered and the polymer to provide stabilization of the agent and retention of activity upon delivery; ii) rate of polymer degradation and thus agent release profile; iii) surface characteristics and targeting capabilities via chemical modification; and iv) particle porosity. Polymeric particles may be prepared using single and double emulsion solvent evaporation, spray drying, solvent extraction, solvent evaporation, phase separation, simple and complex coacervation, interfacial polymerization, jet milling and other methods well known to those of ordinary skill in the art. Particles may be made using methods for making microspheres or microcapsules known in the art.

If desired, the pharmaceutical formulations can include one or more additional agents, such as mucoactive or mucolytic agents, surfactants, antibiotics, antivirals, antihistamines, cough suppressants, bronchodilators, anti-inflammatory agents, steroids, vaccines, adjuvants, expectorants, macromolecules, therapeutics that are helpful for chronic maintenance of CF.

Examples of suitable mucoactive or mucolytic agents include MUC5AC and MUC5B mucins, DNA-ase, N-acetylcysteine (NAC), cysteine, nacystelyn, dornase alfa, gelsolin, heparin, heparin sulfate, P2Y2 agonists (*e.g*. UTP, INS365), hypertonic saline, and mannitol.

Suitable surfactants include L-alpha-phosphatidylcholine dipalmitoyl ("DPPC"), diphosphatidyl glycerol (DPPG), 1,2-Dipalmitoyl-sn-glycero-3-phospho-L-serine (DPPS), 1,2-Dipalmitoyl-sn-glycero-3-phosphocholine (DSPC), 1,2-Distearoyl-sn-glycero-3-phosphoethanolamine (DSPE), 1-palmitoyl-2-oleoylphosphatidylcholine (POPC), fatty alcohols, polyoxyethylene-9-lauryl ether, surface active fatty, acids, sorbitan trioleate (Span 85), glycocholate, surfactin, poloxomers, sorbitan fatty acid esters, tyloxapol, phospholipids, and alkylated sugars.

If desired, the salt formulation can contain an antibiotic. For example, salt formulations for treating bacterial pneumonia or VAT, can further comprise an antibiotic, such as a macrolide (*e.g*., azithromycin, clarithromycin and erythromycin), a tetracycline (*e.g*., doxycycline, tigecycline), a fluoroquinolone (*e.g*., gemifloxacin, levofloxacin, ciprofloxacin and mocifloxacin), a cephalosporin (*e.g*., ceftriaxone, defotaxime, ceftazidime, cefepime), a penicillin (*e.g*., amoxicillin, amoxicillin with clavulanate, ampicillin, piperacillin, and ticarcillin) optionally with a β-lactamase inhibitor (*e.g*., sulbactam, tazobactam and clavulanic acid), such as ampicillin-sulbactam, piperacillin-tazobactam and ticarcillin with clavulanate, an aminoglycoside (*e.g*., amikacin, arbekacin, gentamicin, kanamycin, neomycin, netilmicin, paromomycin, rhodostreptomycin, streptomycin, tobramycin, and apramycin), a penem or carbapenem (*e.g*. doripenem, ertapenem, imipenem and meropenem), a monobactam (*e.g*., aztreonam), an oxazolidinone (*e.g*., linezolid), vancomycin, glycopeptide antibiotics (*e.g*. telavancin), tuberculosis-mycobacterium antibiotics and the like.

If desired, the salt formulation can contain an agent for treating infections with mycobacteria, such as *Mycobacterium tuberculosis.* Suitable agents for treating infections with mycobacteria (*e.g*., *M. tuberculosis)* include an aminoglycoside (*e.g*. capreomycin, kanamycin, streptomycin), a fluoroquinolone (*e.g*. ciprofloxacin, levofloxacin, moxifloxacin), isozianid and isozianid analogs (*e.g*. ethionamide), aminosalicylate, cycloserine, diarylquinoline, ethambutol, pyrazinamide, protionamide, rifampin, and the like.

If desired, the salt formulation can contain a suitable antiviral agent, such as oseltamivir, zanamavir amantidine or rimantadine, ribavirin, gancyclovir, valgancyclovir, foscavir, Cytogam® (Cytomegalovirus Immune Globulin), pleconaril, rupintrivir, palivizumab, motavizumab, cytarabine, docosanol, denotivir, cidofovir, and acyclovir. Salt formulation can contain a suitable antiinfluenza agent, such as zanamivir, oseltamivir, amantadine, or rimantadine.

Suitable antihistamines include clemastine, asalastine, loratadine, fexofenadine and the like.

Suitable cough suppressants include benzonatate, benproperine, clobutinal, diphenhydramine, dextromethorphan, dibunate, fedrilate, glaucine, oxalamine, piperidione, opiods such as codine and the like.

Suitable brochodilators include short-acting beta₂ agonists, long-acting beta₂ agonists (LABA), long-acting muscarinic anagonists (LAMA), combinations of LABAs and LAMAs, methylxanthines, and the like. Suitable short-active beta2 agonists include albuterol, epinephrine, pirbuterol, levalbuterol, metaproteronol, maxair, and the like. Suitable LABAs include salmeterol, formoterol and isomers (e.g. arformoterol), clenbuterol, tulobuterol, vilanterol (Revolair™), indacaterol, and the like. Examples of LAMAs include tiotroprium, glycopyrrolate, aclidinium, ipratropium and the like. Examples of combinations of LABAs and LAMAs include indacaterol with glycopyrrolate, indacaterol with tiotropium, and the like. Examples of methylxanthine include theophylline, and the like.

Suitable anti-inflammatory agents include leukotriene inhibitors, PDE4 inhibitors, other anti-inflammatory agents, and the like. Suitable leukotriene inhibitors include montelukast (cystinyl leukotriene inhibitors), masilukast, zafirleukast (leukotriene D4 and E4 receptor inhibitors), zileuton (5-lipoxygenase inhibitors), and the like. Suitable PDE4 inhibitors include cilomilast, roflumilast, and the like. Other anti-inflammatory agents include omalizumab (anti IgE immunoglobulin), IL-13 and IL-13 receptor inhibitors (such as AMG-317, MILR1444A, CAT-354, QAX576, IMA-638, Anrukinzumab, IMA-026, MK-6105,DOM-0910 and the like), IL-4 and IL-4 receptor inhibitors (such as Pitrakinra, AER-003,AIR-645, APG-201, DOM-0919 and the like) IL-1 inhibitors such as canakinumab, CRTh2 receptor antagonists such as AZD 1981 (from AstraZeneca), neutrophil elastase inhibitor such as AZD9668 (from AstraZeneca), P38 kinase inhibitor such as losmapimed, and the like.

Suitable steroids include corticosteroids, combinations of corticosteroids and LABAs, combinations of corticosteroids and LAMAs, and the like. Suitable corticosteroids include budesonide, fluticasone, flunisolide, triamcinolone, beclomethasone, mometasone, ciclesonide, dexamethasone, and the like. Combinations of corticosteroids and LABAs include salmeterol with fluticasone, formoterol with budesonide, formoterol with fluticasone, formoterol with mometasone, indacaterol with mometasone, and the like.

Suitable expectorants include guaifenesin, guaiacolculfonate, ammonium chloride, potassium iodide, tyloxapol, antimony pentasulfide and the like.

Suitable vaccines such as nasally inhaled influenza vaccines and the like.

Suitable macromolecules include proteins and large peptides, polysaccharides and oligosaccharides, and DNA and RNA nucleic acid molecules and their analogs having therapeutic, prophylactic or diagnostic activities. Proteins can include antibodies such as monoclonal antibody. Nucleic acid molecules include genes, antisense molecules such as siRNAs that bind to complementary DNA, RNA, or ribosomes to inhibit transcription or translation.

Selected therapeutics that are helpful for chronic maintenance of cystic fibrosis include antibiotics/macrolide antibiotics, bronchodilators, inhaled LABAs, and agents to promote airway secretion clearance. Suitable examples of antibiotics/macrolide antibiotics include tobramycin, azithromycin, ciprofloxacin, colistin, and the like. Suitable examples of bronchodilators include inhaled short-acting beta₂ agonists such as albuterol, and the like. Suitable examples of inhaled LABAs include salmeterol, formoterol, and the like. Suitable examples of agents to promote airway secretion clearance include dornase alfa, hypertonic saline, and the like.

Some non-limiting examples of the pharmaceutical formulations of the invention can be found in Table 1

| Table 1. | | | | | | | |
|---|---|---|---|---|---|---|---|
| Liquid formulations of Calcium Chloride | | | | | | | |
| Formulation # | Tonicity (1X = isotonic) | CaCl₂ (% w/v) | CaCl₂ (M) | NaCl (% w/v) | NaCl (M) | Ratio of Ca:Na (molar) | |
| 1 | 8X | 9.0 | 0.81 | 0.90 | 0.15 | 5.4 | |
| 2 | 11X | 13 | 1.2 | 0.90 | 0.15 | 8.0 | |
| 3 | 0.5X | 0.59 | 0.053 | 0.040 | 0.0070 | 7.6 | |
| 4 | 1X | 1.2 | 0.11 | 0.080 | 0.013 | 8.5 | |
| 5 | 2X | 2.4 | 0.21 | 0.16 | 0.027 | 7.8 | |
| 6 | 4X | 4.7 | 0.42 | 0.31 | 0.053 | 7.9 | |
| 7 | 8X | 9.4 | 0.85 | 0.62 | 0.11 | 7.7 | |
| | | | | | | | |

| Liquid formulations of Calcium Lactate | | | | | | | |
|---|---|---|---|---|---|---|---|
| Formulation | Tonicity (1X = isotonic) | Ca-lactate (%) | Ca-lactate (M) | NaCl (%) | NaCl (M) | | |
| 8 | 0.5X | 1.4 | 0.065 | 0.048 | 0.0082 | 7.9 | |
| 9 | 1X | 2.9 | 0.13 | 0.10 | 0.016 | 8.1 | |
| 10 | 2X | 5.7 | 0.26 | 0.19 | 0.033 | 7.9 | |
| 11 | 3X | 9.0 | 0.41 | 0.30 | 0.052 | 7.9 | |
| 12 | 4X | 11 | 0.52 | 0.38 | 0.065 | 8.0 | |
| 13 | 8X | 23 | 1.0 | 0.77 | 0.13 | 7.7 | |
| | | | | | | | |

| Powder formulations | | | | | | | |
|---|---|---|---|---|---|---|---|
| Formulation # | Formulation composition | | | | | | |
| | Excipient | Excipient (wt %) | Calcium salt | Calcium salt (wt %) | Sodium salt | Sodium salt (wt %) | Ratio of Ca:Na (molar) |
| 14 | 10.0 | Calcium chloride | 83.3 | Sodium sulfate | 6.7 | 10.0 | 8 |
| 15 | 67.6 | Calcium chloride | 30.0 | Sodium sulfate | 2.4 | 67.6 | 8 |
| 16 | 10.0 | Calcium chloride | 82.0 | Sodium citrate | 8.0 | 10.0 | 8 |
| 17 | 67.1 | Calcium chloride | 30.0 | Sodium citrate | 2.9 | 67.1 | 8 |
| 18 | 10.0 | Calcium lactate | 87.1 | Sodium chloride | 2.9 | 10.0 | 8 |
| 19 | 69.0 | Calcium lactate | 30.0 | Sodium chloride | 1.0 | 69.0 | 8 |
| 20 | 10.0 | Calcium chloride | 77.6 | Sodium sulfate | 12.4 | 10.0 | 4 |
| 21 | 65.2 | Calcium chloride | 30.0 | Sodium sulfate | 4.8 | 65.2 | 4 |
| 22 | 10.0 | Calcium chloride | 75.4 | Sodium citrate | 14.6 | 10.0 | 4 |
| 23 | 64.2 | Calcium chloride | 30.0 | Sodium citrate | 5.8 | 64.2 | 4 |
| 24 | 10.0 | Calcium lactate | 84.4 | Sodium Chloride | 5.6 | 10.0 | 4 |
| 25 | 68.0 | Calcium lactate | 30.0 | Sodium chloride | 2.0 | 68.0 | 4 |

### 4. Treatment of pulmonary Diseases or Respiratory Tract Infections

Also described are methods for treatment (including prophylactic treatment) of pulmonary diseases, such as asthma (*e.g*., allergic/atopic, childhood, late-onset, cough-variant, or chronic obstructive), airway hyperresponsiveness, allergic rhinitis (seasonal or non-seasonal), brochiectasis, chronic bronchitis, emphysema, chronic obstructive pulmonary disease, cystic fibrosis, early life wheezing, and the like, and for the treatment (including prophylactic treatment) of acute exacerbations of these chronic diseases, such as exaccibations caused by a viral infection (e.g., influenza virus such as Influenza virus A or Influenza virus B, parainfluenza virus, respiratory syncytial virus, rhinovirus, adenovirus, metapneumovirus, coxsackie virus, echo virus, corona virus, herpes simplex vins, cytomegalovirus, and the like), bacterial infections (*e.g*., *Streptococcus pneumoniae*, which is commonly referred to as pneumococcus, *Staphylococcus aureus, Streptococcus agalactiae, Streptococcus pyogenes, Haemophilus influenzae, Haemophilus parainfluenzae, Klebsiella pneumoniae, Escherichia coli, Pseudomonas aeruginosa, Moraxella catarrhalis, Chlamydophila pneumoniae, Mycoplasma pneumoniae, Legionella pneumophila, Serratia marcescens, Burkholderia cepacia, Burkholderia pseudomallei, Bacillus anthracis, Bacillus cereus, Stenotrophomonas maltophilia,* a bacterium from the citrobacter family, a bacterium from the ecinetobacter family, *Mycobacterium tuberculosis, Bordetella pertussis,* and the like), fungal infections (e.g., *Histoplasma capsulatum, Cryptococcus neoformans, Pneumocystisjiroveci, Coccidioides immitis, Candida albicans, Pneumocystis jirovecii* (which causes pneumocystis pneumonia (PCP), also called pneumocystosis), and the like), parasitic infections (e.g., *Toxoplasma gondii, Strongyloides stercoralis,* and the like), or environmental allergens and irritants (e.g., aeroallergens, including pollen, house-dust mites, animal dander such as cat dander, molds, cockroaches, airborne particulates, and the like).

Also described are methods for treatment (including prophylactic treatment) of infectious diseases of the respiratory tract, *e.g.*, viral infections or bacterial infections of the respiratory tract.

Exemplary respiratory tract infections include pneumonia (including community-acquired pneumonia, nosocomial pneumonia (hospital-acquired pneumonia, HAP; health-care associated pneumonia, HCAP), ventilator-associated pneumonia (VAP)), ventilator-associated trachebronchitis (VAT), bronchitis, croup (e.g., postintebation croup, and infectious croup), tuberculosis, influenza, common cold, and viral infections (e.g., influenza virus (e.g., Influenza virus A, Influenza virus B), parainfluenza virus (e.g., hPIV-1, hPIV-2, hPIV-3, hPIV-4), respiratory syncytial virus, rhinovirus, adenovirus, metapneumovirus, coxsackie virus, echo virus, corona virus, herpes simplex virus, cytomegalovirus, enterovirus, SARS-coronavirus, and smallpox, and the like), bacterial infections (e.g., *Streptococcus pneumoniae,* which is commonly referred to as pneumococcus, *Staphylococcus aureus, Streptococcus pyogenes, Streptococcus agalactiae, Haemophilus influenzae, Haemophilus parainfluenzae, Klebsiella pneumoniae, Escherichia coli, Pseudomonas aeruginosa, Moraxella catarrhalis, Chlamydophila pneumoniae, Mycoplasma pneumoniae, Legionella pneumophila, Serratia marcescens, Mycobacterium tuberculosis, Bordetella pertussis, Burkholderia cepacia, Burkholderia pseudomallei, Bacillus anthracis, Bacillus cereus, Stenotrophomonas maltophilia,* a bacterium from the citrobacter family, a bacterium from the ceinetobacter family, and the like), fungal infections (e.g., *Histoplasma capsulatum, Cryptococcus neoformans, Pneumocystisjiroveci, Coccidioides immitis, Candida albicans, Pneumocystis jirovecii* (which causes pneumocystis pneumonia (PCP), also called pneumocystosis), and the like), or parasitic infections (e.g., *Toxoplasma gondii, Strongyloides stercoralis,* and the like).

In certain embodiments, the respiratory tract infection is a bacterial infection, such as bacterial pneumonia. In certain embodiments, the bacterial infection is caused by a bacterium selected from the group consisting of *Streptococcus pneumoniae* (also referred to as pneumococcus), *Staphylococcus aureus, Streptococcus agalactiae, Streptococcus pyogenes, Haemophilus influenzae, Haemophilus parainfluenzae, Klebsiella pneumoniae, Escherichia coli, Pseudomonas aeruginosa, Moraxella catarrhalis, Chlamydophila pneumoniae, Mycoplasma pneumoniae, Legionella pneumophila, Serratia marcescens, Burkholderia cepacia, Burkholderia pseudomallei, Bacillus anthracis, Bacillus cereus, Bordatella pertussis, Stenotrophomonas maltophilia,* a bacterium from the citrobacter family, a bacterium from the ecinetobacter family, and *Mycobacterium tuberculosis.*

In certain embodiments, the respiratory tract infection is a viral infection, such as influenza or viral pneumonia. In certain embodiments, the viral infection is caused by a virus selected from the group consisting of influenza virus (e.g., Influenza virus A, Influenza virus B), respiratory syncytial virus, adenovirus, metapneumovirus, cytomegalovirus, parainfluenza virus (e.g., hPIV-1, hPIV-2, hPIV-3, hPIV-4), rhinovirus, adenovirus, coxsackie virus, echo virus, corona virus, herpes simplex virus, SARS-coronavirus, and smallpox.

In certain embodiments, the respiratory tract infection is a fungal infection. In certain embodiments, the fungal infection is caused by a fungus selected from the group consisting of *Histoplasma capsulatum, Cryptococcus neoformans, Pneumocystis jiroveci, Coccidioides immitis, Candida albicans,* and *Pneumocystis jirovecii* (which causes pneumocystis pneumonia (PCP), also called pneumocystosis).

In certain embodiments, the respiratory tract infection is a parasitic infection. In certain embodiments, the parasitic infection is caused by a parasite selected from the group consisting of *Toxoplasma gondii* and *Strongyloides stercoralis.*

Also described is a method for treating (including prophylactically treating) an individual with a pulmonary disease (e.g., an individual having a pulmonary disease, exhibiting symptoms of a pulmonary disease, or susceptible to a pulmonary disease), comprising administering to the respiratory tract of the individual an effective amount of a pharmaceutical formulation comprising a calcium salt and a sodium salt as active ingredients, wherein the ratio of Ca⁺² to Na⁺ is from about 4:1 (mole:mole) to about 16:1 (mole:mole).

Also described is a method for treating (including prophylactically treating) an acute exacerbation of a chronic pulmonary disease in an individual, comprising administering to the respiratory tract of the individual in need thereof (e.g., an individual having an acute exacerbation of a pulmonary disease, exhibiting symptoms of an acute exacerbation of a pulmonary disease, or susceptible to an acute exacerbation of a pulmonary disease) an effective amount of a pharmaceutical formulation comprising a calcium salt and a sodium salt as active ingredients, wherein the ratio of Ca⁺² to Na⁺ is from about 4:1 (mole:mole) to about 16:1 (mole:mole). Exemplary pulmonary diseases include asthma (*e.g.*, allergic/atopic, childhood, late-onset, cough-variant, or chronic obstructive), airway hyperresponsiveness, allergic rhinitis (seasonal or non-seasonal), bronchiectasis, chronic bronchitis, emphysema, chronic obstructive pulmonary disease, cystic fibrosis, early life wheezing, and the like.

In certain embodiment, influenza is caused by either the influenza A or the influenza B virus.

In certain embodiments, an influenza-like illness is caused by RSV, rhinovirus, adenovirus, parainfluenza, human coronaviruses (including the virus that causes severe acute respiratory syndrome) and metapneumovirus.

In certain embodiments, ventilator associate pneumonia (VAP), ventilator associated tracheobronchitis (VAT), or hospital acquired pneumonia (HAP), is caused by *pneumoniae, S. pneumoniae, S.aureus, non-typeable Haemophilus influenzae (*NTHI*)*, *psuedominas aeruginosa, Acinetobacter spp., E coli, Candida spp (*a fungus*)*, *Serratia, Enterobacter spp,* and *Stenotrophomonas.* Alternatively, VAP or VAT can be caused by Gram-positive or Gram-negative bacteria associated with causing pneumonia.

In certain embodiments, community associated pneumonia (CAP) is caused by at least one of the following bacteria: *Moraxella catarralis, Mycoplasma pneumoniae, Chlamydophilia pneumonia, or Chlamydia pneumoniae, strep pneumonia, Haemophilus influenzae, chlamydophia, mycoplasma,and Legionella.* Alternatively, or in addition to the previously mentioned bacteria, CAP may also be cause by at least one of the following fungi: Coccidiomycosis, histoplasmosis, and cryptococcocus. Alternatively, CAP can be caused by Gram-positive or Gram-negative bacteria associated with causing pneumonia.

In certain embodiments, an acute exacerbation of a patient with asthma is caused by an upper respiratory tract viral infection or Gram-positive or Gram-negative bacteria associated with pneumonia, including CAP. Alternatively, or in addition, an acute exacerbation can be caused by allergens or environmental factors such as house dust mites, Ova, or pollen. An acute exacerbation of a patient with COPD is caused by the same causes as for asthma, and additionally by *Haemophilus influenzae, pneumococcus,* and *moraxella.* Mild exacerbations of CF can be caused by all of the above, in addition to the opportunitistic bacterial pathogens, such as *Pseudomonas aeruginosa, Burkholderia cepacia, Burkholderia pseudomallei,* and the like, that characterize CF airway colonization, and also by atypical *mycobacteria* and *Stenotrophomonas.*

An effective amount of a pharmaceutical formulation as described herein can be administered to the respiratory tract of an individual (*e.g.*, a mammal, such as a human or other primate, or domesticated animal, such as pigs, cows, sheep, chickens) in need thereof. Preferably, the pharmaceutical formulation can be administered by inhalation of an aerosol.

The pharmaceutical formulations may be administered to alter the biophysical and/or biological properties of the mucosal lining of the respiratory tract (*e.g.*, the airway lining fluid) and underlying tissue (*e.g.*, respiratory tract epithelium). These properties include, for example, gelation at the mucus surface, surface tension of the mucosal lining, surface elasticity and/or viscosity of the mucosal lining, bulk elasticity and/or viscosity of the mucosal lining, airway hydration, or ciliary beat.

Described herein is: a method for treating (including prophylactically treating) an individual with a bacterial infection of the respiratory tract, an individual exhibiting symptoms of a bacterial infection of the respiratory tract, or an individual at risk of contracting a bacterial infection of the respiratory tract, comprising administering to the respiratory tract of the individual an effective amount of a pharmaceutical formulation as described herein.

In certain embodiments, the individual being treated is infected, or at risk of being infected by a bacterium selected from the group consisting of *Streptococcus pneumoniae, Staphylococcus aureus, Streptococcus agalactiae, Streptococcus pyogenes, haemophilus influenzae, Klebsiella pneumoniae, Escherichia coli, Pseudonionas aeruginosa, Moraxella catarrhalis, Chlamydophila pneumoniae, Mycoplasma pneumoniae,* and *Legionella pneumophila,* all of which could cause pneumonia. In particular embodiments, the individual is infected, or at risk of being infected, by *Streptococcus pneumoniae, Klebsiella pneumoniae* or *Pseudomonas aeruginosa.* In a more particular embodiment, the individual is infected, or at risk of being infected, by *Streptococcus pneumoniae.*

Also described is a method for treating (including prophylactically treating) an individual with a viral infection of the respiratory tract, an individual exhibiting symptoms of a viral infection of the respiratory tract, or an individual at risk of contracting a viral infection of the respiratory tract, comprising administering to the respiratory tract of the individual an effective amount of a pharmaceutical formulation as described herein.

In some embodiments, the individual being treated is infected, or at risk of being infected, by a virus selected from the group consisting of influenza virus (e.g., Influenza virus A, Influenza virus B), respiratory syncytial virus, adenovirus, metapneumovirus, cytomegalovirus, parainfluenza virus (*e.g.*, hPIV-1, hPIV-2, hPIV-3, hPIV-4), rhinovirus, coronaviruses (*e.g.*, SARS-coronavirus), poxviruses (*e.g.*, smallpox), enterovirus, and herpes simplex virus.

An individual at risk of a respiratory tract infection by a pathogen (*e.g.*, a bacterium, a virus) can be prophylactically treated by administering an effective amount of a pharmaceutical formulation as described herein. The method can be used to prevent or to decrease the rate or incidence of infection by a pathogen (*e.g.*, a bacterium, a virus) that causes a respiratory tract infection.

Generally, individuals are at risk for infection by a pathogen (*e.g.*, a virus, a bacterium) that causes a respiratory tract infection when they are exposed to such a pathogen more frequently than the general population, or have a diminished capacity to resist infection. Individuals who are at risk for such an infection include, for example, health care workers, individuals who are immunosuppressed (*e.g.*, medically, due to other infections, or for other reasons), elderly and young (*e.g.*, infants) individuals, and caregivers and family members of infected persons.

In exemplary embodiments, the pharmaceutical formulation used for treating (including prophylactically treating) a respiratory tract infection comprises a calcium salt and a sodium salt, wherein the ratio of Ca⁺² to Na⁺ is about 8:1 (mole:mole), or about 16:1.(mole:mole).

Also described is a method for treating (including prophylactically treating) an individual with a pulmonary disease (e.g., an individual having a pulmonary disease, exhibiting symptoms of a pulmonary disease, or susceptible to a pulmonary disease), comprising administering to the respiratory tract of the individual an effective amount of a pharmaceutical formulation as described herein. Exemplary pulmonary diseases that can be treated include asthma (*e.g.*, allergic/atopic, childhood, late-onset, cough-variant, or chronic obstructive), early life wheezing, allergic rhinitis (seasonal or non-seasonal), airway hyperresponsiveness, bronchiectasis, chronic bronchitis, emphysema, chronic obstructive pulmonary disease, cystic fibrosis and the like.

Also described is a method for treating (including prophylactically treating) an acute exacerbation of a chronic pulmonary disease in an individual, comprising administering to the respiratory tract of the individual in need thereof (e.g., an individual having an acute exacerbation of a pulmonary disease, exhibiting symptoms of an acute exacerbation of a pulmonary disease, or susceptible to an acute exacerbation of a pulmonary disease) an effective amount of a pharmaceutical formulation as described herein. Exemplary pulmonary diseases that can be treated include asthma (e.g., allergic/atopic, childhood, late-onset, cough-variant, or chronic obstructive), early life wheezing, allergic rhinitis (seasonal or non-seasonal), airway hyperresponsiveness, bronchiectasis, chronic bronchitis, emphysema, chronic obstructive pulmonary disease, cystic fibrosis and the like.

Generally, an individual is susceptible to a pulmonary disease or an acute exacerbation of a pulmonary disease when it has been determined, e.g., by individual or family history, or genetic testing, to have a significantly higher than normal probability of having the onset or recurrence of a pulmonary disease or an acute exacerbation of a pulmonary disease or they are exposed to a pulmonary or repiratory pathogen more frequently then the general population, or have a diminished capacity to resist infection. Individuals who are at risk for infection by such a pathogen include, for example, health care workers, individuals who are immunosuppressed (*e.g.*, medically, due to other infections, or for other reasons), patients in an intensive care unit, elderly and young (*e.g.*, infants) individuals, individuals with chronic underlying respiratory disease (e.g., asthma, chronic bronchitis, chronic obstructive pulmonary disease, cystic fibrosis) individuals who have had surgery or traumatic injury, and care givers and family members of infected persons.

The pharmaceutical formulations administered in accordance with any of the methods of treatment described herein may be hypotonic, isotonic or hypertonic as desired. For example, the pharmaceutical formulation administered in accordance with any method described herein can have about 0.1X tonicity, about 0.25X tonicity, about 0.5X tonicity, about 1X tonicity, about 2X tonicity, about 3X tonicity, about 4X tonicity, about 5X tonicity, about 6X tonicity, about 7X tonicity, about 8X tonicity, about 9X tonicity, about 10X tonicity, at least about 1X tonicity, at least about 2X tonicity, at least about 3X tonicity, at least about 4X tonicity, at least about 5X tonicity, at least about 6X tonicity, at least about 7X tonicity, at least about 8X tonicity, at least about 9X tonicity, at least about 10X tonicity, between about 0.1X to about 1X, between about 0.1X to about 0.5X, between about 0.5X to about 2X, between about 1X to about 4X, between about 1X to about 2X, between about 2X to about 10X. or between about 4X to about 8X.

### 5. Reducing Contagion

Also described are methods for reducing contagion (*e.g.*, reducing transmission or spread) of a respiratory tract infection (*e.g.*, a viral infection, a bacterial infection) comprising administering to the respiratory tract (*e.g.*, lungs, nasal cavity) of an individual infected with a pathogen that causes a respiratory tract infection, exhibiting symptoms of a respiratory tract infection, or at risk of contracting a respiratory tract infection by a pathogen (*e.g.*, a bacterium, a virus), an effective amount of a pharmaceutical formulation as described herein.

In some embodiments, the individual may have a respiratory tract infection caused by a bacterium, exhibit symptoms of a respiratory tract infection caused by a bacterium, or be at risk for such an infection as described herein. For example, the individual may be infected, or at risk of being infected, by a bacterium selected from the group consisting of *Streptococcus pneumoniae, Staphylococcus aureus, Streptococcus pyogenes, Streptococcus agalactiae, Haemophilus influenzae, Klebsiella pneumoniae, Escherichia coli, Pseudomonas aeruginosa, Moraxella catarrhalis, Chlamydophila pneumoniae, Mycoplasma pneumoniae, Legionella pneumophila, Bacillus anthracis, Mycobacterium tuberculosis, Bordetella pertussis, Burkholderia cepacia, Burkholderia pseudomallei, Bacillus anthracis, Bacillus cereus, stenotrophomonas maltophilia,* a bacterium from the citrobacter family, and a bacterium from the ecinetobacter family. In particular embodiments, the individual is infected by or at risk of infection by *Streptococcus pneumoniae, Klebsiella pneumoniae* or *Pseudomonas aeruginosa.* In a more particular embodiment, the individual is infected, or at risk of being infected, by *Streptococcus pneumoniae.*

In other embodiments, the individual may be infected by or at risk of being infected by, a virus selected from the group consisting of influenza virus (e.g., Influenza virus A, Influenza virus B), respiratory syncytial virus, adenovirus, metapneumovirus, cytomegalovirus, parainfluenza virus (e.g., hPIV-1, hPIV-2, hPIV-3, hPIV-4), rhinovirus, coronoaviruses (*e.g.*, SARS-coronavirus), poxviruses (*e.g.*, smallpox), enterovirus, and herpes simplex virus..

In exemplary embodiments, the pharmaceutical formulation used for reducing contagion/spread of a respiratory tract infection comprises a calcium salt and a sodium salt, wherein the ratio of Ca⁺² to Na⁺ is about 8:1 (mole:mole), or about 16:1 (mole:mole).

The pharmaceutical formulations administered in accordance with any of the methods of reducing contagion described herein may be hypotonic, isotonic or hypertonic as desired. For example, the pharmaceutical formulation administered in accordance with any method described herein can have about 0.1X tonicity, about 0.25X tonicity, about 0.5X tonicity, about 1X tonicity, about 2X tonicity, about 3X tonicity, about 4X tonicity, about 5X tonicity, about 6X tonicity, about 7X tonicity, about 8X tonicity, about 9X tonicity, about 10X tonicity, at least about 1X tonicity, at least about 2X tonicity, at least about 3X tonicity, at least about 4X tonicity, at least about 5X tonicity, at least about 6X tonicity, at least about 7X tonicity, at least about 8X tonicity, at least about 9X tonicity, at least about 10X tonicity, between about 0.1X to about 1X, between about 0.1X to about 0.5X, between about 0.5X to about 2X, between about 1X to about 4X, between about 1X to about 2X, between about 2X to about 10X, or between about 4X to about 8X.

### 6. Administering the pharmaceutical formulations

The pharmaceutical formulations as described herein are intended for administration to the respiratory tract (e.g., to the mucosal surface of the respiratory tract), and can be administered in any suitable form, such as a solution, a suspension, a spray, a mist, a foam, a gel, a vapor, droplets, particles, or a dry powder form. Preferably, a pharmaceutical formulation as described herein is aerosolized for administration. Many suitable methods and devices that are conventional and well-known in the art can be used to aerosolize the formulation.

For example, the pharmaceutical formulation can be aerosolized for administration via the oral airways using a metered dose inhaler (*e.g.*, a pressurized metered dose inhalers (pMDI) including HFA propellant, or a non-HFA propellant) with or without a spacer or holding chamber, a nebulizer, an atomizer, a continuous sprayer, an oral spray or a dry powder inhaler (DPI). The pharmaceutical formulation can be aerosolized for administration via the nasal airways using a nasal pump or sprayer, a metered dose inhaler (*e.g.*, a pressurized metered dose inhaler (pMDI) including HFA propellant, or a non-HFA propellant) with or without a spacer or holding chamber, a nebulizer with or without a nasal adapter or prongs, an atomizer, a continuous sprayer, or a dry powder inhaler (DPI). The pharmaceutical formulation can also be delivered to the nasal mucosal surface via, for example, nasal wash and to the oral mucosal surfaces via, for example, an oral wash. The pharmaceutical formulation can be delivered to the mucosal surfaces of the sinuses via, for example, nebulizers with nasal adapters and nasal nebulizers with oscillating or pulsatile airflows.

The geometry of the airways is an important consideration when selecting a suitable method for producing and delivering aerosols of pharmaceutical formulations to the lungs. The lungs are designed to entrap particles of foreign matter that are breathed in, such as dust. There are three basic mechanisms of deposition: impaction, sedimentation, and Brownian motion (J. M. Padfield. 1987. In: D. Ganderton & T. Jones eds. Drug Delivery to the Respiratory Tract, Ellis Harwood, Chicherster, U.K.). Impaction occurs when particles are unable to stay within the air stream, particularly at airway branches. Impacted particles are adsorbed onto the mucus layer covering bronchial walls and eventually cleared from the lungs by mucociliary action. Impaction in the upper airways mostly occurs with particles over 5 µm in aerodynamic diameter. Smaller particles (those less than about 3 µm in aerodynamic diameter) tend to stay within the air stream and be advected deep into the lungs by sedimentation. Sedimentation often occurs in the lower respiratory system where airflow is slower. Very small particles (those less than about 0.6 µm) can deposit by Brownian motion.

For administration, a suitable method (*e.g.*, nebulization, dry powder inhaler) is selected to produce aerosols with the appropriate particle size for preferential delivery to the desired region of the respiratory tract, such as the deep lung (generally particles between about 0.6 microns and 5 microns in diameter), the upper airway (generally particles of about 3 microns or larger diameter), or the deep lung and the upper airway.

An effective amount of a pharmaceutical formulation as described herein is administered to an individual in need thereof, such as an individual who has a respiratory tract infection, who is exhibiting symptoms of a respiratory tract infection, or who is at risk of contracting a respiratory tract infection. Individuals who are hospitalized, and particularly those who are ventilated, are at risk for infection by pathogens that cause a respiratory tract infection.

An "effective amount" is an amount that is sufficient to achieve the desired therapeutic or prophylactic effect, such as an amount sufficient to reduce symptoms of infection (e.g., fever, coughing, sneezing, nasal discharge, diarrhea, and the like), to reduce time to recovery, to reduce pathogens in an individual, to inhibit pathogens passing through the lung mucus or airway lining fluid, to decrease the incidence or rate of infection with pathogens that cause infection of the respiratory tract, and/or to decrease the shedding of exhaled particles containing pathogens that cause a respiratory tract infection. More specifically, an effective amount can be an amount that is sufficient to increase surface and/or bulk viscoelasticy of the respiratory tract mucus (e.g., airway lining fluid), to increase gelation of the respiratory tract mucus (e.g., at the surface and/or bulk gelation), to increase surface tension of the respiratory tract mucus, to increase elasticity of the respiratory tract mucus (e.g., surface elasticity and/or bulk elasticity), to increase surface viscosity of the respiratory tract mucus (e.g., surface viscosity and/or bulk viscosity), to reduce the amount of exhaled particles, to reduce pathogen (e.g., bacterial, viral) burden (e.g. by reducing pathogen uptake into body, killing or inactivating existing pathogen found in the body, increasing clearance of pathogen from body such as by increasing airway hydration, increasing ciliary beat, increasing mucociliary clearance (Groth et al, Thorax, 43(5):360-365 (1988) and the like). Because the pharmaceutical formulations are administered to the respiratory tract, generally by inhalation, the dose that is administered is related to the composition of the formulation (*e.g.*, calcium salt concentration, sodium salt concentration), the rate and efficiency of aerosolization (*e.g.*, nebulization rate and efficiency), and the time of exposure (*e.g.*, nebulization time). For example, substantially equivalent doses can be administered using a concentrated liquid pharmaceutical formulation and a short (*e.g.*, 5 minutes) nebulization time, or using a dilute liquid pharmaceutical formulation and a long (*e.g.*, 30 minutes or more) nebulization time, or using a dry powder formulation and a dry powder inhaler. The clinician of ordinary skill can determine appropriate dosage based on these considerations and other factors, for example, the individual's age, sensitivity, tolerance and overall well-being. A pharmaceutical formulation as described herein can be administered in a single dose or multiple doses as indicated.

Since the amount of cation provided can vary depending upon the particular calcium salt or sodium salt selected, dosing can be based on the desired amount of cation to be delivered to the lung. For example, one mole of calcium chloride (CaCl₂) dissociates to provide one mole of Ca²⁺, but one mole of tricalcium phosphate (Ca₃(PO₄)₂) can provide three moles of Ca²⁺.

Generally, an effective amount of a pharmaceutical formulation will deliver a dose of about 0.001 mg Ca⁺²/kg body weight/dose to about 2 mg Ca⁺²/kg body weight/dose, about 0.002 mg Ca⁺²/kg body weight/dose to about 2 mg Ca⁺²/kg body weight/dose, about 0.005 mg Ca⁺²/kg body weight/dose to about 2 mg Ca⁺²/kg body weight/dose, about 0.01 mg Ca⁺²/kg body weight/dose to about 2 mg Ca⁺²/kg body weight/dose, about 0.01 mg Ca⁺²/kg body weight/dose to about 60 mg Ca⁺²/kg body weight/dose, about 0.01 mg Ca⁺²/kg body weight/dose to about 50 mg Ca⁺²/kg body weight/dose, about 0.01 mg Ca⁺²/kg body weight/dose to about 40 mg Ca⁺²/kg body weight/dose, about 0.01 mg Ca⁺²/kg body weight/dose to about 30 mg Ca⁺²/kg body weight/dose, about 0.01 mg Ca⁺²/kg body weight/dose to about 20 mg Ca⁺²/kg body weight/dose, about 0.01 mg Ca⁺²/kg body weight/dose to about 10 mg Ca⁺²/kg body weight/dose, about 0.01 mg Ca⁺²/kg body weight/dose to about 5 mg Ca⁺²/kg body weight/dose, about 0.01 mg Ca⁺² /kg body weight/dose to about 2 mg Ca⁺²/kg body weight/dose, about 0.02 mg Ca⁺²/kg body weight/dose to about 2 mg Ca⁺²/kg body weight/dose, about 0.03 mg Ca⁺²/kg body weight/dose to about 2 mg Ca⁺²/kg body weight/dose, about 0.04 mg Ca⁺²/kg body weight/dose to about 2 mg Ca⁺²/kg body weight/dose, about 0.05 mg Ca⁺²/kg body weight/dose to about 2 mg Ca⁺²/kg body weight/dose, about 0.1 mg Ca⁺²/kg body weight/dose to about 2 mg Ca⁺²/kg body weight/dose, about 0.1 mg Ca⁺²/kg body weight/dose to about 1 mg Ca⁺²/kg body weight/dose, about 0.1 mg Ca⁺²/kg body weight/dose to about 0.5 mg Ca⁺²/kg body weight/dose, about 0.2 mg Ca⁺²/kg body weight/dose to about 0.5 mg Ca⁺²/kg body weight/dose, about 0.18 mg Ca⁺²/kg body weight/dose, about 0.001 mg Ca⁺²/kg body weight/dose, about 0.005 mg Ca⁺²/kg body weight/dose, about 0.01 mg Ca⁺²/kg body weight/dose, about 0.02 mg Ca⁺²/kg body weight/dose, or about 0.5 mg Ca⁺²/kg body weight/dose.

The amount of sodium delivered to the respiratory tract depends on the amount of calcium salt present in the formulation, and the desired calcium:sodium ratio. In some embodiments, the amount of sodium delivered to the respiratory tract is about 0.001 mg Na⁺/kg body weight/dose to about 10 mg Na⁺/kg body weight/dose, or about 0.01 mg Na⁺/kg body weight/dose to about 10 mg Na⁺/kg body weight/dose, or about 0.1 mg Na⁺/kg body weight/dose to about 10 mg Na⁺/kg body weight/dose, or about 1.0 mg Na⁺/kg body weight/dose to about 10 mg Na⁺/kg body weight/dose, or about 0.001 mg Na⁺/kg body weight/dose to about 1 mg Na⁺/kg body weight/dose, or about 0.01 mg Na⁺/kg body weight/dose to about 1 mg Na⁺/kg body weight/dose, about 0.1 mg Na⁺/kg body weight/dose to about 1 mg Na⁺/kg body weight/dose, about 0.2 mg Na⁺/kg body weight/dose to about 0.8 mg Na⁺/kg body weight/dose, about 0.3 mg Na⁺/kg body weight/dose to about 0.7 mg Na⁺/kg body weight/dose, or about 0.4 mg Na⁺/kg body weight/dose to about 0.6 mg Na⁺/kg body weight/dose.

In some embodiments the amount of calcium delivered to the respiratory tract (*e.g.*, lungs, respiratory airway) is about 0.001 mg Ca⁺²/kg body weight/dose to about 2 mg Ca⁺²/kg body weight/dose, about 0.002 mg Ca⁺²/kg body weight/dose to about 2 mg Ca⁺²/kg body weight/dose, about 0.005 mg Ca⁺²/kg body weight/dose to about 2 mg Ca⁺²/kg body weight/dose, about 0.01 mg Ca⁺²/kg body weight/dose to about 2 mg Ca⁺²/kg body weight/dose, about 0.01 mg Ca⁺²/kg body weight/dose to about 60 mg Ca⁺²/kg body weight/dose, about 0.01 mg Ca⁺²/kg body weight/dose to about 50 mg Ca⁺²/kg body weight/dose, about 0.01 mg Ca⁺²/kg body weight/dose to about 40 mg Ca⁺²/kg body weight/dose, about 0.01 mg Ca⁺²/kg body weight/dose to about 30 mg Ca⁺²/kg body weight/dose, about 0.01 mg Ca⁺²/kg body weight/dose to about 20 mg Ca⁺²/kg body weight/dose, about 0.01 mg Ca⁺²/kg body weight/dose to about 10 mg Ca⁺²/kg body weight/dose, about 0.01 mg Ca⁺²/kg body weight/dose to about 5 mg Ca⁺²/kg body weight/dose, about 0.01 mg Ca⁺²/kg body weight/dose to about 2 mg Ca⁺²/kg body weight/dose, about 0.02 mg Ca⁺²/kg body weight/dose to about 2 mg Ca⁺²/kg body weight/dose, about 0.03 mg Ca⁺²/kg body weight/dose to about 2 mg Ca⁺²/kg body weight/dose, about 0.04 mg Ca⁺²/kg body weight/dose to about 2 mg Ca⁺²/kg body weight/dose, about 0.05 mg Ca⁺²/kg body weight/dose to about 2 mg Ca⁺²/kg body weight/dose, about 0.1 mg Ca⁺²/kg body weight/dose to about 2 mg Ca⁺²/kg body weight/dose, about 0.1 mg Ca⁺²/kg body weight/dose to about 1 mg Ca⁺²/kg body weight/dose, about 0.1 mg Ca⁺²/kg body weight/dose to about 0.5 mg Ca⁺²/kg body weight/dose, about 0.2 mg Ca⁺²/kg body weight/dose to about 0.5 mg Ca⁺²/kg body weight/dose, about 0.18 mg Ca⁺²/kg body weight/dose, about 0.001 mg Ca⁺²/kg body weight/dose, about 0.005 mg Ca⁺²/kg body weight/dose, about 0.01 mg Ca⁺²/kg body weight/dose, about 0.02 mg Ca⁺²/kg body weight/dose, or about 0.5 mg Ca⁺²/kg body weight/dose.

In other embodiments the amount of calcium delivered to the upper respiratory tract (e.g., nasal cavity) is of about 0.001 mg Ca⁺²/kg body weight/dose to about 2 mg Ca⁺²/kg body weight/dose, about 0.002 mg Ca⁺²/kg body weight/dose to about 2 mg Ca⁺²/kg body weight/dose, about 0.005 mg Ca⁺²/kg body weight/dose to about 2 mg Ca⁺²/kg body weight/dose, about 0.01 mg Ca⁺²/kg body weight/dose to about 2 mg Ca⁺²/kg body weight/dose, about 0.01 mg Ca⁺²/kg body weight/dose to about 60 mg Ca⁺²/kg body weight/dose, about 0.01 mg Ca⁺²/kg body weight/dose to about 50 mg Ca⁺²/kg body weight/dose, about 0.01 mg Ca⁺²/kg body weight/dose to about 40 mg Ca⁺²/kg body weight/dose, about 0.01 mg Ca⁺²/kg body weight/dose to about 30 mg Ca⁺²/kg body weight/dose, about 0.01 mg Ca⁺²/kg body weight/dose to about 20 mg Ca⁺²/kg body weight/dose, about 0.01 mg Ca⁺²/kg body weight/dose to about 10 mg Ca⁺²/kg body weight/dose, about 0.01 mg Ca⁺²/kg body weight/dose to about 5 mg Ca⁺²/kg body weight/dose, about 0.01 mg Ca⁺²/kg body weight/dose to about 2 mg Ca⁺²/kg body weight/dose, about 0.02 mg Ca⁺²/kg body weight/dose to about 2 mg Ca⁺²/kg body weight/dose, about 0.03 mg Ca⁺²/kg body weight/dose to about 2 mg Ca⁺²/kg body weight/dose, about 0.04 mg Ca⁺²/kg body weight/dose to about 2 mg Ca⁺²/kg body weight/dose, about 0.05 mg Ca⁺²/kg body weight/dose to about 2 mg Ca⁺²/kg body weight/dose, about 0.1 mg Ca⁺²/kg body weight/dose to about 2 mg Ca⁺²/kg body weight/dose, about 0.1 mg Ca⁺²/kg body weight/dose to about 1 mg Ca⁺²/kg body weight/dose, about 0.1 mg Ca⁺²/kg body weight/dose to about 0.5 mg Ca+²/kg body weight/dose, about 0.2 mg Ca⁺²/kg body weight/dose to about 0.5 mg Ca⁺²/kg body weight/dose, about 0.18 mg Ca⁺²/kg body weight/dose, about 0.001 mg Ca⁺²/kg body weight/dose, about 0.005 mg Ca⁺²/kg body weight/dose, about 0.01 mg Ca⁺²/kg body weight/dose, about 0.02 mg Ca⁺²/kg body weight/dose, or about 0.5 mg Ca⁺²/kg body weight/dose.

In some embodiments the amount of sodium delivered to the respiratory tract (e.g., lungs, respiratory airway) is about 0.001 mg Na⁺/kg body weight/dose to about 10 mg Na⁺/kg body weight/dose, or about 0.01 mg Na⁺/kg body weight/dose to about 10 mg Na⁺/kg body weight/dose, or about 0.1 mg Na⁺/kg body weight/dose to about 10 mg Na⁺/kg body weight/dose, or about 1.0 mg Na⁺/kg body weight/dose to about 10 mg Na⁺/kg body weight/dose, or about 0.001 mg Na⁺/kg body weight/dose to about 1 mg Na⁺/kg body weight/dose, or about 0.01 mg Na⁺/kg body weight/dose to about 1 mg Na⁺/kg body weight/dose, about 0.1 mg Na⁺/kg body weight/dose to about 1 mg Na⁺/kg body weight/dose, about 0.2 mg Na⁺/kg body weight/dose to about 0.8 mg Na⁺/kg body weight/dose, about 0.3 mg Na⁺/kg body weight/dose to about 0.7 mg Na⁺/kg body weight/dose, or about 0.4 mg Na⁺/kg body weight/dose to about 0.6 mg Na⁺/kg body weight/dose.

In other embodiments the amount of sodium delivered to the upper respiratory tract (e.g., nasal cavity) is about 0.001 mg Na⁺/kg body weight/dose to about 10 mg Na⁺/kg body weight/dose, or about 0.01 mg Na⁺/kg body weight/dose to about 10 mg Na⁺/kg body weight/dose, or about 0.1 mg Na⁺/kg body weight/dose to about 10 mg Na⁺/kg body weight/dose, or about 1.0 mg Na⁺/kg body weight/dose to about 10 mg Na⁺/kg body weight/dose, or about 0.001 mg Na⁺/kg body weight/dose to about 1 mg Na⁺/kg body weight/dose, or about 0.01 mg Na⁺/kg body weight/dose to about 1 mg Na⁺/kg body weight/dose, about 0.1 mg Na⁺/kg body weight/dose to about 1 mg Na⁺/kg body weight/dose, about 0.2 mg Na⁺/kg body weight/dose to about 0.8 mg Na⁺/kg body weight/dose, about 0.3 mg Na⁺/kg body weight/dose to about 0.7 mg Na⁺/kg body weight/dose, or about 0.4 mg Na⁺/kg body weight/dose to about 0.6 mg Na⁺/kg body weight/dose.

The pharmaceutical formulations can be delivered to the upper respiratory tract (*e.g.*, nasal passages, nasal cavity, throat, pharynx), respiratory airways (*e.g.*, larynx, tranchea, bronchi, bronchioles) or lungs (*e.g.*, respiratory bronchioles, alveolar ducts, alveolar sacs, alveoli).

Generally, inhalation devices (e.g., DPIs) should be able to deliver a therapeutically effective amount of a composition described herein in a single inhalation. In some cases however, to achieve the intended therapeutic results, multiple inhalations and/or frequent administration may be required. For example, the desired dose of calcium and sodium may be delivered with two or more inhalations (e.g., from a capsule-type or blister-type inhaler). Preferably, each dose that is administered to a subject in need thereof contains an effective amount of a composition described herein and is administered using no more than about 4 inhalations. For example, each dose of a composition described herein can be administered in a single inhalation or 2, 3, or 4 inhalations. The compositions are preferably administered in a single, breath-activated step using a breath-activated DPI.

Suitable intervals between doses that provide the desired therapeutic effect can be determined based on the severity of the condition (*e.g.*, infection), overall well being of the subject and the subject's tolerance to the pharmaceutical formulations and other considerations. Based on these and other considerations, a clinician can determine appropriate intervals between doses. Generally, a pharmaceutical formulation is administered once, twice or three times a day, as needed.

If desired or indicated, the pharmaceutical formulation can be administered with one or more other therapeutic agents. The other therapeutic agents can be administered by any suitable route, such as orally, parenterally (*e.g.*, intravenous, intraarterial, intramuscular, or subcutaneous injection), topically, by inhalation (*e.g.*, intrabronchial, intranasal or oral inhalation, intranasal drops), rectally, vaginally, and the like. The pharmaceutical formulation can be administered before, substantially concurrently with, or subsequent to administration of the other therapeutic agent. Preferably, the pharmaceutical formulation and the other therapeutic agent are administered so as to provide substantial overlap of their pharmacologic activities.

### EXEMPLIFICATION

The invention now being generally described, it will be more readily understood by reference to the following examples, which are included merely for purposes of illustration of certain aspects and embodiments of the present invention, and are not intended to limit the invention.

### Example 1: Calcium to Sodium Ratios

In this example, formulations comprising calcium and sodium at different ratios were tested in order to determine whether certain concentrations and ratios provide superior therapeutic activities (e.g., reduction in viral titers). Formulations with Ca⁺²:Na⁺ ratios from about 4:1 to about 16:1 of (mole:mole) were identified as having superior therapeutic activities.

### Methods:

A cell culture model of influenza infection was used to study the effects of different nebulized solutions on viral infection. Calu-3 cells were cultured on permeable membranes (12mm Transwells; 0.4µm pore size, Coming Lowell, MA) until confluent (membrane is fully covered with cells) and air-liquid interface (ALI) cultures were established by removing the apical media and culturing at 37°C / 5% CO₂. Cells were cultured for >2 weeks ALI before each experiment.

Prior to each experiment the apical surface of each Transwell was washed 3X with PBS. Cells were subsequently exposed to nebulized formulations using a Sedimentation chamber and Series 8900 nebulizers. Immediately after exposure, the basolateral media (media on the bottom side of the Transwell) was replaced with fresh media. Triplicate wells were exposed to each formulation in each test. A second cell culture plate was exposed to the same formulations to quantify the delivery of total salt or calcium to cells.

One hour after exposure, cells were infected with 10µL of Influenza A/WSN/33/1 at a multiplicity of infection of 0.1-0.01 (0.1-0.01 virions per cell). Four hours after aerosol treatment, the apical surfaces were washed to remove excess formulation and unattached virus and cells were cultured for an additional 20 hours at 37°C plus 5% CO₂. The next day (24 hours after infection) virus released onto the apical surface of infected cells was collected in culture media or PBS and the concentration of virus in the apical wash was quantified by TCID₅₀ (50% Tissue Culture Infectious Dose) assay. The TCID₅₀ assay is a standard endpoint dilution assay that is used to quantify virus in a sample.

### Results:

### (a) Calcium to Sodium Ratios

Previous *in vitro* studies have shown that formulations that contain a sodium salt and a calcium salt can suppress exhaled particles. To determine whether certain concentrations and/or ratios of the two cations and/or salts could provide superior therapeutic activities, a panel of formulations comprising various concentrations of calcium chloride and sodium chloride were tested for their respective activities in reducing Influenza viral replication. The total salt concentration in the formulations tested ranged from 0.115M to 1.3M. An untreated (Air) control was included in each study and viral titers (Log TCID₅₀/mL) from treated wells were normalized to the air control from each study.

Figure 1 shows the effect of various sodium and calcium concentrations on viral replication. To normalize the data from all experiments, the rate of change of viral replication in each treated condition, as compared to the zero salt condition, was determined for each experiment. Assuming that viral replication proceeds according to the equation, Nₜ=N₀*e^{(kt)} (wherein No is the original number of viral particles at time 0, k is replication rate constant, and Nₜ is the number of viral particles at time t), then t(k-k') can be determined where t is fixed (all experiments end at t) and k' is the new replication rate constant (e.g., the replication rate constant in the presence of a particular salt formulation). Through this analysis, a higher value represents a smaller k', and thus a greater decrease in the rate of viral infectivity (or greater activity in reducing viral infection). A contour plot was generated with JMP analysis software. The X-axis depicts increasing CaCl₂ concentration and the Y-axis depicts increasing NaCl concentration. Each dot represents a formulation tested with at least three wells per test. Reduced viral infectivity is shown by increasing darkness (*i.e.*, higher numbers and darker shades represent formulations with greater effects on reducing Influenza viral replication).

As the concentration of calcium was increased, a corresponding decrease in viral infectivity was observed, consistent with previous studies that demonstrated a dose-responsive effect of calcium in this model. In addition, these studies highlighted a range of calcium to sodium concentrations that exhibited superior antiviral activities. The calcium concentrations ranged from 0.575M to 1.15M and the sodium concentrations ranged from 0.075M to 0.3M, which resulted in calcium to sodium ratios between about 4:1 and about 16:1 (mole:mole), and a middle point of 8:1 (mole:mole).

### (b) Formulations with an 8:1 ratio or a 16: 1 ratio of calcium to sodium reduced viral infectivity in a dose responsive manner

From the testing of a number of different formulations comprising calcium and sodium at different ratios, formulations with Ca⁺²:Na⁺ ratios at 8:1 or 16:1 (mole:mole) were identified as particularly effective in reducing viral replication. To determine if formulations at these ratios were effective over a range of salt concentrations, dose response studies were performed. Concentrated solutions of calcium chloride were formulated at an 8:1 ratio or a 16:1 ratio to sodium chloride and subsequently diluted in water. Through this approach, the ratios of calcium to sodium were held constant but the concentration of each ion was reduced. Formulations of both ratios reduced viral infection in a dose dependent manner (Figures 2A, 2B) and exhibited comparable effects to one another at similar concentrations of calcium. In both cases, the doses were increased by increasing tonicity of the liquid formulation, while keeping dosing time constant.

### Example 2: Calcium:Sodium Formulations Reduced the Infectivity of Multiple Influenza Strains in vitro

In this example, the *in vitro* therapeutic activities of calcium:sodium_formulations (with a Ca²⁺:Na⁺ ratio at 8:1 (mole:mole)) were tested using multiple strains of Influenza viruses. The formulations were varied in tonicity (either 0.5X, 2X or 8X the tonicity of an isotonic solution). The formulations were shown to effectively reduce the infectivity of a broad array of influenza viruses.

### Methods:

Calu-3 cells were cultured on permeable membranes (12mm Transwells; 0.4µm pore size, Coming Lowell, MA) until confluent (membrane is fully covered with cells) and air-liquid interface (ALI) cultures were established by removing the apical media and culturing at 37°C / 5% CO₂. Cells were cultured for >2 weeks at ALI before each experiment. Normal human bronchial epithelial (NHBE) cells were seeded at passage 2 on permeable membranes (12mm Millicell, 0.4 µm pore size; Millipore Billerica, MA) and incubated (37°C, 5% CO₂, 95% RH) until confluent under liquid-covered culture conditions. Once confluent, the apical media was removed and ALI cultures were established. Cells were cultured for ≥4 weeks ALI prior to each experiment.

Prior to each experiment the apical surface of each cell type was washed 3X with PBS. Cells were subsequently exposed to nebulized formulations with a Sedimentation chamber and Series 8900 nebulizers. In experiments performed on Calu-3 cells, Transwells were exposed to calcium:sodium formulations at an 8:1 molar ratio (0.5X, 2X, and 8X tonicity; 1X = isotonic) in triplicate.

Experiments performed on NHBE cells involved exposing Millicells to nebulized calcium:sodium formulations at an 8:1 molar ratio (0.5X, 2X, or 8X tonicity; 1X = isotonic). Immediately after exposure, the basolateral media (media on the bottom side of the Transwell or Millicell) was replaced with fresh media.

For experiments performed on both Calu-3 cells and NHBE cells, triplicate wells for each cell type were exposed to each formulation in each test. A second cell culture plate was exposed to the same formulations to quantify the delivery of total salt or calcium to cells. One hour after exposure, cells were infected with 5-10µL of Influenza virus at a multiplicity of infection of 0.1-0.001 (0.1-0.001 virions per cell). Four hours after aerosol treatment, the apical surfaces were washed to remove excess formulation and unattached virus and cells were cultured for an additional 20 hours at 37°C plus 5% CO₂. The next day, viruses that were released onto the apical surface of infected cells were collected in culture media or PBS and the concentration of viruses in the apical wash were quantified by TCID₅₀ (50% Tissue Culture Infectious Dose) assay (a standard endpoint dilution assay used to quantify viral titers). The influenza strains used in this study are listed in Table 2.

**Table 2. Influenza strains used in Example 2**

| **Influenza A H1N1** | **Influenza A H3N2** | **Influenza B** |
|---|---|---|
| A/WSN/33 | A/Aichi/2/68 | B/Mass/3/66 |
| A/Puerto Rico/8/34 | A/Panama/2007/99 | |
| A/FM/1/47 | A/Port Chalmers/1/73 | |
| A/Weiss/43 | | |
| A/Swine/IA/40776/92 | | |

### Results

### (a) Calcium:Sodium Formulations Reduced the Infectivity of an H3N2 Influenza Strain in vitro.

Example 1 shows that the calcium:sodium formulations effectively reduced viral replication of Influenza strain A/WSN/33/1 (H1N1). In this example, we tested the activities of the formulations on a different Influenza strain, A/Panama/2007/99 (H3N2)_Further, because Calu-3 cells are immortalized cells of lung origin, we also tested primary normal human bronchial epithelial (NHBE) cell cultures. These cultures are multicellular and are minimally passaged *in vitro* prior to testing.

NHBE cells were exposed to calcium:sodium formulations (8:1 ratio of Ca:Na) at different tonicities, and then infected with Influenza A/Panama/2007/99. Because NHBE cells are primary cells, the cultures were subject to donor-to-donor variability that was not present in Calu-3 cultures. To account for this variability, NHBE cultures from four different donors were tested. Treatment of NHBE cell cultures resulted in reduced influenza titers in all donors tested, with a maximal reduction of greater than 100-fold in each case (Figure 3). Thus, the calcium:sodium formulations were effective in reducing viral infectivity in Calu-3 cells as well as primary NHBE cell cultures, further supporting the therapeutic values of the formulations to treat (including prophylactically treat) influenza infection.

### (b) Calcium:Sodium Formulations Reduced the Infectivity of Multiple Influenza Strains in vitro.

Calcium:sodium formulations at an 8:1 Ca²⁺: Na⁺ molar ratio were shown to reduce the infectivity of multiple H1N1 influenza strains and one H3N2 influenza strain (Examples 1 and 2(a), additional data not shown). We then tested two additional H3N2 strains and an influenza B strain. Additionally, we tested four additional H1N1 strains, including one isolated from swine. We find that the calcium:sodium formulations reduced the infectivity of all viruses tested in a dose responsive manner (Figure 4). The greatest reduction in viral titer was observed using the 8X formulation, which reduced viral titers between 32- to 12,589- fold depending on the specific viral strain being tested. Thus, the calcium:sodium formulations (Ca²⁺:Na⁺ molar ratio at 8: 1) can effectively reduce the infectivity of a broad array of influenza viruses.

### Example 3: In vivo Therapeutic Activities of Calcium:Sodium Formulations in Reducing Infectivity of Influenza Virus in a Ferret Influenza Model

In this example, the *in vivo* therapeutic activities of two liquid formulations with a Ca²⁺:Na⁺ ratio at 8:1 (mole:mole) were tested using a ferret model of influenza. The formulations were hypertonic (either 4X or 8X the tonicity of an isotonic solution). The treatments were shown to improve the clinical course of influenza infection and dampen the inflammatory response to influenza infection in ferrets.

### Methods:

The ferret model of influenza is a standard model for the evaluation of influenza vaccines or antivirals. Using this model we tested the therapeutic activities of FORMULATION A and two 8:1 molar ratio formulations that had enhanced activity against influenza replication *in vitro.* The formulations tested are shown in Table 3. Control ferrets were exposed to inhalation grade water for the same duration (6.5 minutes) and under the same exposure conditions. Aerosol formulations were generated from two PariLC Sprint nebulizers and ferrets were exposed to nebulized formulations using a FlowPast exposure system (TSE systems). Ferrets were dosed 1 hour before infection, 4 hours after infection and then BID for 6 days until the termination of the study. Nasal wash samples were collected once daily beginning on day 1 of the study and body temperatures and body weights were determined twice a day beginning on day 0 of the study. The number of inflammatory cells and the viral titer in nasal wash samples were determined

**Table 3: Calcium:sodium formulations that were tested on a ferret model of influenza**

| Formulation | Calcium chloride concentration (M) | Sodium chloride concentration (M) | Ratio Ca:Na | Delivered dose (mg CaCl₂/kg)* |
|---|---|---|---|---|
| FORMULATION A | 0.116 | 0.15 | 1:1.3 | 0.56 |
| 4X | 0.424 | 0.053 | 8:1 | 1.99 |
| 8X | 0.849 | 0.106 | 8:1 | 3.44 |

| | | | | |
|---|---|---|---|---|
| * The delivered dose was determined from measurements made from the sample port of the nose-only exposure system and the calcium concentration was determined by HPLC methods. | | | | |

### Results:

### (a) The calcium:sodium formulations prevented the onset and reduced the severity of fever

Compared to control ferrets, 4X and 8X treated ferrets exhibited a delayed onset of fever and a reduced peak body temperature, as compared to the control group (Figure 5). At the time of peak fever (36 hours post infection), the 4X treatment groups exhibited significantly reduced body temperatures as compared to the control group (mean increase of 3.4°C in the control group compared to 0.4°C in the 4X group; p<0.05 and p<0.01, respectively Mann-Whitney U test; Figure 5). The 8X treatment group also exhibited reduced body temperatures as compared to control animals (mean increase 1.45 °C). These data show that the calcium:sodium formulations reduced the severity and delayed the onset of fever following influenza infection in ferrets.

### (b) The calcium:sodium formulations prevented body weight loss

Control ferrets lost weight more rapidly and exhibited a greater percentage of body weight loss 48 hours post-infection, as compared to treated animals (Figure 6). The differences in body weight loss between the 4X and 8X groups and the control group were statistically significant (4.0% weight loss in the control group, as compared to 3.1% and 2.4% in the 4X and 8X groups, respectively; Figure 6). The data also showed a dose-responsive prevention of weight loss as the 8X treated animals lost the least amount of weight.

### (c) The calcium:sodium formulations reduced nasal inflammatory cell counts

Influenza infection in the upper airways is associated with an infiltration of inflammatory cells. This inflammation is also a primary cause of the clinical symptoms associated with infection. To determine if treatment with calcium:sodium formulations could reduce inflammation following influenza infection, the number of inflammatory cells in each nasal wash from control or treated ferrets was determined. Inflammatory cell counts were significantly lower in the treated groups as compared to the control group over the course of the study (Figure 7; p<0.0001 Two-way ANOVA). The total numbers of inflammatory cells recovered from 4X and 8X treated ferrets were significantly lower than that of control ferrets at 72 hours post infection (p<0.01 for 4X; p<0.001 for the 8X treatment; Mann-Whitney U test). Furthermore, the 4X and 8X treatments resulted in a statistically significant difference in inflammatory cell counts at 120 hours post infection (p<0.05 Mann-Whitney U test). In contrast, FORMULATION A was less effective in reducing the number of inflammatory cells at 120 hours post infection (Figure 7C). The results show that at higher doses, inflammation may be inhibited for longer periods of time. Collectively, these data demonstrate that the calcium:sodium formulations reduced the clinical symptoms and dampened the inflammatory response associated with influenza infection.

These *in vivo* data showed that the calcium:sodium formulations treatments improve the clinical course of influenza infection and dampened the inflammatory response to influenza infection in ferrets (e.g., reduced body weight loss, reduced inflammatory cell counts, etc.).

Influenza infections are common respiratory tract infections that are typically treated by Influenza specific antiviral drugs/agents or prophylactically treated by Influenza vaccines. Antivirals are limited in that a specific diagnosis of Influenza is required and treatment must start soon after infection or symptom onset to be effective. Additionally, there are a number of respiratory tract infections, collectively called influenza-like illness (ILI) caused by other viruses that cause a similar symptomology as Influenza infection, which make a precise diagnosis difficult. Influenza vaccines have only limited coverage each year as they target only a subset of Influenza viruses that circulate among the population in a given year. For both antivirals and vaccines, the emergence of resistant viruses refractory to treatment is also of concern.

Formulations described herein could be administered at the first onset of symptoms without a precise diagnosis of the infectious agent and/or be administered prophylactically to subjects that are at risk of exposure to respiratory tract infections.

### Example 4: Activities of Calcium:Sodium Formulations in Reducing hPIV Infectivity

In this example, the effectiveness of 8:1 calcium:sodium formulations in reducing human parainfluenza virus (hPIV) infectivity was examined. The data showed that the 8:1 calcium:sodium formulations reduced hPIV-3 viral replication in both Calu-3 and NHBE cells.

Human parainfluenza virus is a single stranded RNA enveloped virus distinct from Influenza. These viruses are 150-300nm in diameter and are covered in hemagglutinin-neuraminidase (HN) spikes and fusion proteins. Unlike influenza virus, the genome is non-segmented and following attachment of the virus to the target cell via HN tetramers the virus is believed to fuse directly with the plasma membrane (Moscona, A. (2005). "Entry of parainfluenza virus into cells as a target for interrupting childhood respiratory disease." J Clin Invest 115(7): 1688-98). hPIV-3 is associated with upper and lower respiratory tract disease and frequently a cause of influenza-like illness (ILI).

### Methods:

A cell culture model of human parainfluenza virus 3 (hPIV-3) infection was used to study the effects of different nebulized solutions on viral infection. Calu-3 cells were cultured on permeable membranes (12mm Transwells; 0.4µm pore size, Coming Lowell, MA) until confluent (membrane is fully covered with cells) and air-liquid interface (ALI) cultures were established by removing the apical media and culturing at 37°C / 5% CO₂. Cells were cultured for >2 weeks at ALI before each experiment. Normal human bronchial epithelial (NHBE) cells were seeded at passage 2 on permeable membranes (12mm Millicell, 0.4 µm pore size; Millipore Billerica, MA) and incubated (37°C, 5% CO₂, 95% RH) until confluent under liquid-covered culture conditions. Once confluent, the apical media was removed and ALI cultures were established. Cells were cultured for ≥4 weeks ALI prior to each experiment. Prior to each experiment the apical surface of each cell type was washed 3X with PBS.

Cells were subsequently exposed to nebulized formulations using a Sedimentation chamber and Series 8900 nebulizers. In experiments performed on Calu-3 cells, Transwells were exposed to nebulized Ca²⁺:Na⁺ formulations at an 8:1 molar ratio (0.5X, 2X, and 8X) in triplicate. Experiments performed on NHBE cells involved exposing Millicells to nebulized Ca²⁺:Na⁺ formulations at an 8:1 molar ratio (0.5X, 2X, and 8X) in duplicate. Immediately after exposure, the basolateral media (media on the bottom side of the Transwell) was replaced with fresh media. Triplicate wells were exposed to each formulation in each test. A second cell culture plate was exposed to the same formulations to quantify the delivery of total salt or calcium to cells. One hour after exposure, cells were infected with 10µL of hPIV-3 (C242 strain) at a multiplicity of infection of 0.3-0.1 (0.3-0.1 virions per cell). Four hours after aerosol treatment, the apical surfaces were washed to remove excess formulation and unattached virus. The next day (24 hours after formulation exposure) virus released onto the apical surface of infected cells was collected in culture media or PBS and the concentration of virus in the apical wash was quantified by TCID₅₀ (50% Tissue Culture Infectious Dose) assay.

### Results:

Previous examples showed that replication of Influenza virus was significantly reduced in a dose responsive manner by calcium:sodium formulations, and identified a preferred Ca⁺²:Na⁺ molar ratio at 8:1. To further examine the therapeutic activities of calcium:sodium formulations in reducing viral titer and to determine the broad spectrum nature of the treatment *in vitro,* the effects of calcium:sodium formulations on human parainfluenza virus 3 (hPIV-3) replication were tested.

To determine whether the calcium:sodium formulations could effectively reduce hPIV-3 infection, Calu-3 cells or NHBE cells were exposed to the 8:1 calcium:sodium formulations at different tonicities (0.5X, 2X, and 8X). Untreated cells were used as controls. Similar to the findings with Influenza, hPIV-3 infection was reduced by the calcium:sodium formulations in a dose responsive manner (Figure 8). In both Calu-3 and NHBE cells, treatment with 8X Ca2⁺:Na⁺ formulation resulted in the greatest decrease in titer as compared to the untreated control (*p<0.001*, as compared to respective untreated control; one-way ANOVA with Tukey's multiple comparison post-test), however all three treatments had a significant impact on infection. The 0.5X formulation reduced hPIV-3 infection by 15.8- and 79.4-fold in Calu-3 and NHBE cells, respectively, and the 2X formulation reduced hPIV-3 infection by 631- and 5011-fold, respectively.

These data extend the findings made with Influenza, and demonstrate that the calcium:sodium formulations are broadly effective in reducing viral infections caused by unrelated viruses.

### Example 5: Activities of Calcium:Sodium Formulations in Reducing Rhinovirus Infectivity

Previous examples showed that replication of Influenza virus and parainfluenza virus (hPIV) was significantly reduced in a dose responsive manner by calcium:sodium formulations, and identified a preferred Ca⁺²:Na⁺ molar ratio at 8:1. To further examine the therapeutic activities of calcium:sodium formulations in reducing viral titer and to determine the broad spectrum nature of the treatment *in vitro*, the effectiveness of 8:1 calcium:sodium formulations in reducing human rhinovirus infectivity was examined. The data showed that the 8:1 calcium:sodium formulations reduced replication of rhinovrius in Calu-3 cells.

Calu-3 cells were exposed to 8X calcium:sodium formulation (8:1 molar ratio of Ca²⁺:Na⁺). Untreated cells were used as controls. Treatment of the cells with the 8X formulation reduced rhinovirus infection by 1.5 Log₁₀ TCID₅₀/mL, as compared to the untreated control (Figure 9; *p<0.01* compared to untreated control; t-test).

These data extend the findings made with Influenza and parainfluenza, and demonstrate that the calcium:sodium formulations are also effective in reducing viral infections caused by non-enveloped viruses such as rhinovirus. Together with the data obtained with calcium:sodium formulations in influenza and parainfluenza models, this result demonstrates that the calcium:sodium formulations are broadly effective in reducing the infectivity of multiple, unrelated respiratory viruses.

### Example 6: Therapeutic Activities of Calcium:Sodium Formulations in Treating Bacterial Infections

In this example, the therapeutic activities of the calcium:sodium formulations in treating bacterial infections were examined using a mouse model. The data showed that the calcium:sodium formulations were effective in treating *Streptococcus pneumoniae* infection in the mouse model.

### Methods:

Bacteria were prepared by growing cultures on tryptic soy agar (TSA) blood plates overnight at 37°C plus 5%CO₂. Single colonies were resuspended to an OD₆₀₀ ~ 0.3 in sterile PBS and subsequently diluted 1:4 in sterile PBS (~2x10⁷ Colony forming units (CFU)/mL). Mice were infected with 50µL of bacterial suspension (~1x10⁶ CFU) by intratracheal instillation while under anesthesia.

C57BL6 mice were exposed to aerosolized liquid formulations in a whole-body exposure system using Pari LC Sprint nebulizers connected to a pie chamber cage that individually holds up to 11 animals. Treatments were performed 2 hours before infection with Serotype 3 *Streptococcus pneumoniae.* Unless otherwise stated, exposure times were 3 minutes in duration. Twenty-four hours after infection mice were euthanized by pentobarbital injection and lungs were collected and homogenized in sterile PBS. Lung homogenate samples were serially diluted in sterile PBS and plated on TSA blood agar plates. CFU were enumerated the following day.

### Results:

### (a) Calcium:sodium formulations (Ca²⁺:Na⁺ at 8:1 molar ratio) reduced bacterial burden in a dose responsive manner

The therapeutic activities of the calcium:sodium formulations was evaluated in the same model and over a wide dose range. With nebulization dosing time held constant, different doses were delivered by using formulations consisting of different concentrations of Ca2⁺:Na⁺ and therefore different tonicities. The 8:1 Ca²⁺:Na⁺ formulations reduced bacterial burden in a dose responsive manner, with the greatest reduction observed at lower doses of calcium (about a 4-fold reduction at a dose of 0.32 mg Ca²⁺/kg and tonicity of 0.5X, and about a 5-fold reduction at a dose of 0.72mg Ca²⁺/kg and tonicity of 1.0X) (Figure 10). Interestingly, these reductions were comparable to the reduction seen for FORMULATION A, however at significantly lower doses. The 2X tonicity formulation, which is equivalent to FORMULATION A in tonicity, had a relatively modest effect on reducing bacterial titers (~1.6 fold reduction) when administered at a dose of 1.58 mg Ca²⁺/kg.

### (b) Increasing dose through longer nebulizations did not significantly affect the therapeutic activities of the calcium:sodium formulations

Figure 10 showed that that calcium:sodium formulations at an 8:1 molar ratio of calcium to sodium reduced the severity of bacterial infections at doses of less than 1.58 mg Ca²⁺/kg. Specifically, while all the formulations tested were effective, the 1X formulation (~0.72 mg Ca²⁺/kg) showed highest therapeutic activity. The study whose results were presented in Figure 10 tested a dose time of 3 minutes. To further examine the effect of dosage, we tested a dose range of Ca²⁺ by increasing the duration of dosing. Animals were treated with a Ca2^{+:}Na+ formulation (1X tonicity = isotonic; 8:1 molar ratio) for different amounts of time (1.5 minutes to 12 minutes). These dose times resulted in Ca²⁺ dosages at approximately 0.36, 0.72, 1.44, and 2.88mg Ca²⁺/kg for the 1.5, 3, 6, and 12 minutes dosing times, respectively. As shown in Figure 11, at the shortest dosing time, no decrease in bacterial titer was observed as compared to control animals (which were dosed 3 minutes with saline), whereas the 3, 6, and 12 minutes doses each reduced bacterial titers to statistically significant levels.

### Example 7: In vivo Therapeutic Activities of Calcium:Sodium Formulations in Reducing Infectivity of Influenza Virus in a Mouse Influenza Model

In this example, the *in vivo* therapeutic activities of four liquid formulations with a Ca²⁺:Na⁺ ratio at 8:1 (mole:mole) were tested using a mouse model of influenza. The formulations were isotonic or hypertonic (1X, 2X, 4X or 8X the tonicity of an isotonic solution). The treatments were shown to improve the clinical course of influenza infection in mice.

### Methods:

Mice (Balb/c) were treated with the indicated formulations beginning 3 hours before infection, 3 hours after infection and then BID for up to 11 days. For influenza infections, mice were lightly anesthetized with ketamine/xylazine and a lethal dose of virus (Influenza A/PR/8) was delivered intranasally. The primary endpoint of the study was animal survival for up to 21 days after infection. Animal body temperatures and body weights were monitored throughout the study. Animals with body temperatures below 95°F were euthanized.

### Results:

Examples 3 and 6 demonstrated that the calcium:sodium formulations described herein were effective in treating bacterial pneumonia in a mouse model and influenza in a ferret model. In the bacterial model, the 1X formulation and doses less than 1.5mg Ca²⁺/kg were found to be most effective. In the ferret influenza model, higher doses were more effective in improving the course of infection. Here we tested the dose ranges of the 1X, 2X, 4X, or 8X formulations, each at a Ca²⁺:Na⁺ molar ratio of 8:1, in a mouse model of influenza.

To study the effects of the calcium:sodium formulations on influenza infection, survival studies were performed. Mice were administered with a lethal dose of influenza virus and treated with the calcium:sodium formulations at different tonicities. The dose time for each formulation was constant, resulting in increased doses of calcium as the tonicity of the formulation was increased. Saline treated animals were used as controls. Figure 12 shows the survival data for each group over time. In this study, 75% of the control animals died before the end of the study on day 21. In contrast, 50% of the 4X and 42.9% of the 8X treated animals died, demonstrating that treatment with 4X and 8X formulations improved animal survival rate.

## Claims

1. A pharmaceutical formulation comprising a calcium salt and a sodium salt, wherein the ratio or Ca⁺² to Na⁺ is from 4:1 (mole:mole) to 16:1 (mole:mole).

2. The pharmaceutical formulation of claim 1, wherein the ratio of Ca⁺² to Na⁺ is from 4:1 (mole:mole) to 12:1 (mole:mole), 4:1 (mole:mole) to 10:1 (mole:mole), or 4: 1 (mole:mole) to 8:1 (mole:mole).

3. The pharmaceutical formulation of claim 1, wherein the ratio of Ca⁺² to Na⁻ is from 4:1 (mole:mole) to 7:1 (mole:mole), or 4:1 (mole:mole) to 6:1 (mole:mole).

4. The pharmaceutical formulation of claim 1, wherein the ratio of Ca⁺² to Na⁺ is 4:1 (mole:mole), or 4.5:1 (mole:mole), or 5:1 (mole:mole), or 5.5:1 (mole:mole), or 6:1 (mole:mole).

5. The pharmaceutical formulation of claim 1, wherein the ratio of Ca⁺² to Na⁺ is 7:1 (mole:mole), 8:1 (mole:mole), 9:1(mole:mole); 10:1 (mole:mole), 11:1 (mole:mole), or 12:1 (mole:mole).

6. The pharmaceutical formulation of claim 1, wherein the ratio of Ca⁺² to Na⁺ is 13:1 (mole:mole), 14:1 (mole:mole 15:1 (mole:mole), or 16:1 (mole:mole).

7. The pharmaceutical formulation of any one of claims 1-6, wherein the pharmaceutical formulation is a liquid formulation, optionally, wherein the Ca²⁺ ion is present in a concentration of from 0.115 M to 1.15 M, or wherein the Ca²⁺ ion is present in a concentration of from 0.575 M to 1.15 M.

8. The pharmaceutical formulation of claim 7, wherein the Na⁺ ion is present in a concentration of from 0.053 M to 0.3 M, or wherein the Na⁺ ion is present in a concentration of from 0.075 M to 0.3 M.

9. The pharmaceutical formulation of claim 7 or 8, wherein the calcium salt is selected from the group consisting of calcium chloride, calcium carbonate, calcium acetate, calcium phosphate, calcium alginate; calcium stearate, calcium sorbate, calcium sulfate, calcium gluconate, calcium lactate and calcium citrate, optionally wherein the calcium salt is calcium lactate, calcium citrate, or calcium sulfate, or wherein the calcium salt is calcium chloride or calcium lactate.

10. The pharmaceutical formulation of any one of claims 7-9, wherein the sodium salt is selected from the group consisting of sodium chloride, sodium acetate, sodium bicarbonate, sodium carbonate, sodium sulfate, sodium stearate, sodium ascorbate, sodium benzoate, sodium biphosphate, sodium phosphate, sodium bisulfite, sodium citrate, sodium berate, sodium gluconate, sodium metasilicate, and sodium lactate, optionally, wherein the sodium salt is sodium chloride, or wherein the sodium salt is sodium lactate, sodium citrate, or sodium sulfate.

11. The pharmaceutical formulation of claim 1-6, wherein the pharmaceutical formulation is a dry powder formulation.

12. The pharmaceutical formulation of claim 11, wherein the calcium salt is present in an amount of from 19.5% to 90% (w/w).

13. The pharmaceutical formulation of claim 11 or 12, wherein the calcium salt is selected from the group consisting of calcium chloride, calcium carbonate, calcium acetate, calcium phosphate, calcium alginate, calcium stearate, calcium sorbate, calcium sulfate, calcium gluconate, calcium lactate and calcium citrate, optionally, wherein the calcium salt is calcium lactate, calcium citrate, calcium sulfate, or calcium chloride.

14. The pharmaceutical formulation of any one of claims 11-13, wherein the sodium salt is selected from the group consisting of sodium chloride, sodium acetate, sodium bicarbonate, sodium carbonate, sodium sulfate, sodium stearate, sodium ascorbate, sodium benzoate, sodium biphosphate, sodium phosphate, sodium bisulfite, sodium citrate, sodium borate, sodium gluconate, sodium metasilicate, and sodium lactate, optionally, wherein the sodium salt is sodium chloride, or wherein the sodium salt is sodium lactate, sodium citrate, or sodium sulfate.

15. The pharmaceutical formulation of any one of claims 1-14, wherein the pharmaceutical formulation is formulated to deliver a Ca⁺² dose of 0.001 mg/kg body weight/dose to 10 mg/kg body weight/dose to the respiratory tract, and/or further comprising an excipient, optionally wherein the excipient is selected from the group consisting of lactose, glycine, alanine, leucine, isolucine, trehalose, dipalmitoylphosphosphatidylcholine (DPPC), diphosphatidyl glycerol (DPPG), 1,2-Dipalmitoyl-sn-glycero-3-phospho-L-serine (DPPS), 1,2-Dipalmitoyl-sn-glycero-3-phosphocholine (DSPC), 1,2-Distearoyl-sn-glycero-3-phosphoethanolamine (DSPE), 1-palmitoyl-2-oleoylphosphatidylcholine (POPC), polyoxyethylene-9-lauryl ether, sorbitan trioleate (Span 85), glycocholate, surfactin, tyloxapol, sodium phosphate, dextran, dextrin, mannitol, maltodextrin, human serum albumin, recombinant human serum albumin, and biodegradable polymers, optionally wherein the excipient is leucine, maltodextrin, or mannitol.

16. The pharmaceutical formulation of any one of claims 1-15, wherein the pharmaceutical formulation is a unit dose formulation.

17. An effective amount of the pharmaceutical formulation of any one of claims 1-16 for use in treating a respiratory tract infection in an individual having a respiratory tract infection, exhibiting symptoms of a respiratory tract infection, or at risk of contracting a respiratory tract infection.

18. An effective amount of the pharmaceutical formulation of any one of claims 1-16 for use in reducing the spread of a respiratory tract infection in an individual having a respiratory tract infection, exhibiting symptoms of a respiratory tract infection, or at risk of contracting a respiratory tract infection,.

19. The pharmaceutical formulation of claim 17 or 18, wherein the respiratory tract infection is influenza, optionally (i) wherein the influenza is caused by Influenza virus A or Influenza virus B, or (ii) wherein the respiratory tract infection is caused by a human parainfluenza virus, optionally wherein the human parainfluenza virus is human parainfluenza virus 3 (hPIV-3), or (iii) wherein the respiratory tract infection is caused by a rhinovirus, or (iv) wherein the respiratory tract infection is caused by a respiratory syncytial virus (RSV), or (v) wherein the respiratory tract infection is pneumonia, optionally wherein the pneumonia is bacterial pneumonia, optionally wherein the bacterial pneumonia is caused by *S. pneumoniae*, or (vi) wherein the respiratory tract infection is a bacterial infection, optionally, wherein the bacterial infection is caused by a bacterium selected from the group consisting of *Streptococcus pneumoniae, Staphylococcus aureus, Streptococcus agalactiae, Streptococcus pyogenes, Haemophilus influenzae, Haemophilus parainfluenzae, Klebsiella pneumoniae, Escherichia coli, Pseudomonas aeruginosa, Moraxella catarrhalis, Chlamydophila pneumoniae, Mycoplasma pneumoniae, Legionella pneumophila, Serratia marcescens, Burkholderia cepacia, Burkholderia pseudomallei, Bacillus anthracis, Bacillus cereus, Bordatella pertussis, Stenotrophomonas maltophilia,* a bacterium from the citrobacter family, a bacterium from the ecinetobacter family, and *Mycobacterium tuberculosis.*

20. An effective amount of the pharmaceutical formulation of any one of claims 1-16 for use in treating a pulmonary disease in an individual having a pulmonary disease, exhibiting symptoms of a pulmonary disease, or susceptible to a pulmonary disease.

21. An effective amount of the pharmaceutical formulation of any one of claims 1-16 for use in treating an acute exacerbation of a pulmonary disease in an individual having an acute exacerbation of a pulmonary disease, exhibiting symptoms of an acute exacerbation of a pulmonary disease, or susceptible to an acute exacerbation of a pulmonary disease,.

22. The pharmaceutical formulation of claims 20 or 21, wherein the pulmonary disease is asthma, airway hyperresponsiveness, allergic rhinitis, seasonal allergic allergy, brochiectasis, chronic bronchitis, emphysema, chronic obstructive pulmonary disease, or cystic fibrosis.

23. The pharmaceutical formulation of claim 21, wherein the acute exacerbation of a chronic pulmonary disease is caused by a viral infection, a bacterial infection, a fungal infection, a parasitic infection, an environmental allergen, or an environmental irritant.

24. The pharmaceutical formulation of any one of claims 1-23, further comprising an additional therapeutic agent.

## Patentansprüche

1. Eine pharmazeutische Zubereitung, ein Calciumsalz und ein Natriumsalz umfassend, wobei das Verhältnis von Ca⁺² zu Na⁺ von 4:1 (Mol:Mol) bis 16:1 (Mol:Mol) ist.

2. Die pharmazeutische Zubereitung nach Anspruch 1, wobei das Verhältnis von Ca⁺² zu Na⁺ von 4:1 (Mol:Mol) bis 12:1 (Mol:Mol), 4:1 (Mol:Mol) bis 10:1 (Mol:Mol) oder 4:1 (Mol:Mol) bis 8:1 (Mol:Mol) ist.

3. Die pharmazeutische Zubereitung nach Anspruch 1, wobei das Verhältnis von Ca⁺² zu Na⁺ von 4:1 (Mol:Mol) bis 7:1 (Mol:Mol) oder 4:1 (Mol:Mol) bis 6:1 (Mol:Mol) ist.

4. Die pharmazeutische Zubereitung nach Anspruch 1, wobei das Verhältnis von Ca⁺² zu Na⁺ ist 4:1 (Mol:Mol) oder 4,5:1 (Mol:Mol) oder 5:1 (Mol:Mol) oder 5,5:1 (Mol:Mol) oder 6:1 (Mol:Mol).

5. Die pharmazeutische Zubereitung nach Anspruch 1, wobei das Verhältnis von Ca⁺² zu Na⁺ ist 7:1 (Mol:Mol), 8:1 (Mol:Mol), 9:1 (Mol:Mol), 10:1 (Mol:Mol), 11:1 (Mol:Mol) oder 12:1 (Mol:Mol).

6. Die pharmazeutische Zubereitung nach Anspruch 1, wobei das Verhältnis von Ca⁺² zu Na⁺ ist 13:1 (Mol:Mol), 14:1 (Mol:Mol), 15:1 (Mol:Mol) oder 16:1 (Mol:Mol).

7. Die pharmazeutische Zubereitung nach einem der Ansprüche 1 bis 6, wobei die pharmazeutische Zubereitung eine flüssige Zubereitung ist, wobei das Ca²⁺-Ion optional in einer Konzentration von 0,115 M bis 1,15 M vorliegt, oder wobei das Ca²⁺-Ion in einer Konzentration von 0,575 M von 1,15 M vorliegt.

8. Die pharmazeutische Zubereitung nach Anspruch 7, wobei das Na⁺-Ion in einer Konzentration von 0,053 M bis 0,3 M vorliegt, oder wobei das Na⁺-Ion in einer Konzentration von 0,075 M bis 0,3 M vorliegt.

9. Die pharmazeutische Zubereitung nach Anspruch 7 oder 8, wobei das Calciumsalz ausgewählt ist aus einer Gruppe bestehend aus Calciumchlorid, Calciumcarbonat, Calciumacetat, Calciumphosphat, Calciumalginat, Calciumstearat, Calciumsorbat, Calciumsulfat, Calcium gluconat, Calciumlactat und Calciumcitrat, wobei optional das Calciumsalz Calciumlactat, Calciumcitrat oder Calciumsulfat ist, oder wobei das Calciumsalz Calciumchlorid oder Calciumlactat ist.

10. Die pharmazeutische Zubereitung nach einem der Ansprüche 7 bis 9, wobei das Natriumsalz ausgewählt ist aus einer Gruppe, bestehend aus Natriumchlorid. Natriumacetat, Natriumbicarbonat, Natriumcarbonat, Natriumsulfat, Natriumstearat, Natriumascorbat, Natriumbenzoat, Natriumbiphosphat, Natriumphosphat, Natriumbisulfit, Natriumcitrat, Natriumborat, Natriumgluconat, Natriummetasilicat und Natriumlactat, wobei optional das Natriumsalz Natriumlactat, Natriumcitrat oder Natriumsulfat ist.

11. Die pharmazeutische Zubereitung nach einem der Ansprüche 1 bis 6, wobei die pharmazeutische Zubereitung eine trockene Pulver-Zubereitung ist.

12. Die pharmazeutische Zubereitung nach Anspruch 11, wobei das Calciumsalz in einer Menge von 19,5 % bis 90 % (w/w) vorliegt.

13. Die pharmazeutische Zubereitung nach Anspruch 11 oder 12, wobei das Calciumsalz ausgewählt ist aus einer Gruppe bestehend aus Calciumchlorid, Calciumcarbonat, Calciumacetat, Calciumphosphat, Calciumalginat, Calciumstearat, Calciumsorbat, Calciumsulfat, Calciumgluconat, Calciumlactat und Calciumcitrat, wobei optional das Calciumsalz Calciumlactat, Calciumcitrat, Calciumsulfat oder Calciumchlorid ist.

14. Die pharmazeutische Zubereitung nach einem der Ansprüche 11 bis 13, wobei das Natriumsalz ausgewählt ist aus einer Gruppe bestehend aus Natriumchlorid,
Natriumacetat, Natriumbicarbonat, Natriumcarbonat, Natriumsulfat, Natriumstearat, Natriumascorbat, Natriumbenzoat, Natriumbiphosphat, Natriumphosphat, Natriumbisulfit, Natriumcitrat, Natriumborat, Natriumgluconat, Natriummetasilicat und Natriumlactat, wobei optional das Natriumsalz Natriumchlorid ist oder wobei das Natriumsalz Natriumlactat, Natriumcitrat oder Natriumsulfat ist.

15. Die pharmazeutische Zubereitung nach einem der Ansprüche 1 bis 14, wobei die pharmazeutische Zubereitung gestaltet ist, um eine Ca⁺²-Dosis von 0,001 mg/kg Körpergewicht/Dosis bis zu 10 mg/kg Körpergewicht/Dosis in die Atemwege abzugeben, und/oder weiterhin einen Arzneiträger umfassend, wobei der Arzneiträger optional ausgewählt ist aus der Gruppe, bestehend aus Lactose, Glycin, Alanin, Leucin, Isoleucin, Trehalose, Dipalmitoylphosphosphatidylcholin (DPPC), Diphosphadylglycerin (DPPG), 1,2-Dipalmitoyl-sn-glycero-3-phospho-L-serin (DPPS), 1,2-Dipalmitoyl-sn-glycero-3-phosphocholin (DSPC), 1,2-Distearoyl-sn-glycero-3-phosphoethanolamin (DSPE), 1-Palmitoyl-2-oleoylphosphatidylcholin (POPC), Polyoxyethylen-9-laurylether, Sorbitantrioleat (Span 85), Glycocholat, Surfactin, Tyloxapol, Natriumphosphat, Dextran, Dextrin, Mannitol, Maltodextrin, Humanalbumin, rekombinantem Humanalbumin und biologisch abbaubaren Polymeren, wobei der Arzneiträger optional Leucin, Maltodextrin oder Mannitol ist.

16. Die pharmazeutische Zubereitung nach einem der Ansprüche 1 bis 15, wobei die pharmazeutische Zubereitung eine Einzeldosis-Zubereitung ist.

17. Eine wirksame Menge der pharmazeutischen Zubereitung nach einem der Ansprüche 1 bis 16 für den Gebrauch in der Behandlung einer Atemwegsinfektion eines Individuums, das eine Atemwegsinfektion hat, das Symptome einer Atemwegsinfektion aufweist oder gefährdet ist, sich eine Atemwegsinfektion zuzuziehen.

18. Eine wirksame Menge der pharmazeutischen Zubereitung nach einem der Ansprüche 1 bis 16 für den Gebrauch in der Reduzierung der Ausbreitung einer Atemwegsinfektion eines Individuums, das eine Atemwegsinfektion hat, das Symptome einer Atemwegsinfektion aufweist oder gefährdet ist, sich eine Atemwegsinfektion zuzuziehen.

19. Die pharmazeutische Zubereitung nach Anspruch 17 oder 18, wobei die Atemwegsinfektion Influenza ist, wobei optional (i) die Influenza verursacht wird durch Influenzavirus A oder Influenzavirus B, oder (ii) wobei die Atemwegsinfektion verursacht wird durch einen humanen Parainfluenzavirus, wobei optional der humane Parainfluenzavirus ein humaner Parainfluenzavirus 3 (hPIV-3) ist, oder (iii) wobei die Atemwegsinfektion verursacht wird durch einen Rhinovirus, oder (iv) wobei die Atemwegsinfektion versursacht wird durch einen Respiratorischen-Synzytial-Virus (RSV), oder (v) wobei die Atemwegsinfektion Lungenentzündung ist, wobei optional die Lungenentzündung eine bakerielle Lungenentzündung ist, wobei die bakterielle Lungenentzündung optional verursacht wird durch *S. pneumoniae*, oder (vi) wobei die Atemwegsinfektion eine bakterielle Infektion ist, wobei optional die bakterielle Infektion verursacht wird durch ein Bakterium, ausgewählt aus der Gruppe, bestehend aus *Streptococcus pneumoniae, Staphylococcus aureus, Streptococcus agalactiae, Streptococcus pyogenes, Haemophilus influenzae, Haemophilus parainfluenzae, Klebsiella pneumoniae, Escherichia coli, Pseudomonas aeruginosa, Moraxella catarrhalis, Chlamydophila pneumoniae, Mycoplasma pneumoniae, Legionella pneumophila, Serratia marcescens, Burkholderia cepacia, Burkholderia pseudomallei, Bacillus anthracis, Bacillus cereus, Bordatella pertussis, Stenotrophomonas maltophilia,* ein Bakterium der Citrobacter-Familie, ein Bakterium der Ecinetobacter-Familie und *Mycobacterium tuberculosis.*

20. Eine wirksame Menge der pharmazeutischen Zubereitung nach einem der Ansprüche 1 bis 16 für den Gebrauch in der Behandlung einer Lungenkrankheit eines Individuums, das eine Lungenkrankheit hat, Symptome einer Lungenkrankheit aufweist oder anfällig für eine Lungenkrankheit ist.

21. Eine wirksame Menge der pharmazeutischen Zubereitung nach einem der Ansprüche 1 bis 16 für den Gebrauch in der Behandlung einer akuten Verschlimmerung einer Lungenkrankheit eines Individuums, das eine akute Verschlimmerung einer Lungenkrankheit hat, Symptome einer akuten Verschlimmerung einer Lungenkrankheit aufweist oder anfällig ist für eine akute Verschlimmerung einer Lungenkrankheit.

22. Die pharmazeutische Zubereitung nach den Ansprüchen 20 oder 21, wobei die Lungenkrankheit Asthma ist, Luftwege-Überreaktion, allergische Rhinitis, saisonale allergische Allergie, Bronchiektasie, chronische Bronchitis, Emphysem, chronische obstruktive Lungenkrankheit oder zystische Fibrose.

23. Die pharmazeutische Zubereitung nach Anspruch 21, wobei die akute Verschlimmerung einer chronischen Lungenkrankheit verursacht wird durch eine virale Infektion, eine bakterielle Infektion, eine Pilzinfektion, eine parasitäre Infektion, ein umweltbedingtes Allergen oder einen umweltbedingten Reizstoff.

24. Die pharmazeutische Zubereitung nach allen Ansprüchen 1 bis 23, weiterhin ein zusätzliches Therapeutikum umfassend.

## Revendications

1. Formulation pharmaceutique comprenant un sel de calcium et un sel de sodium, dans laquelle le rapport Ca²⁺ sur Na⁺ se situe dans la plage allant de 4 : 1 (mole : mole) à 16 : 1 (mole : mole).

2. Formulation pharmaceutique selon la revendication 1, dans laquelle le rapport Ca²⁺ sur Na⁺ se situe dans la plage allant de 4 : 1 (mole : mole) à 12 : 1 (mole : mole), dans celle allant de 4:1 1 (mole : mole) à 10 : 1 (mole : mole), ou dans celle allant de 4 : 1 (mole : mole) à 8 : 1 (mole : mole).

3. Formulation pharmaceutique selon la revendication 1, dans laquelle le rapport Ca²⁺ sur Na⁺ se situe dans la plage allant de 4 : 1 (mole : mole) à 7 : 1 (mole : mole), ou dans celle allant de 4 : 1 (mole : mole) à 6 : 1 (mole : mole).

4. Formulation pharmaceutique selon la revendication 1, dans laquelle le rapport Ca²⁺ sur Na⁺ est de 4 : 1 (mole : mole), ou de 4,5 : 1 (mole : mole), ou de 5 : 1 (mole : mole), ou de 5,5 : 1 (mole : mole), ou de 6 : 1 (mole : mole).

5. Formulation pharmaceutique selon la revendication 1, dans laquelle le rapport Ca²⁺ sur Na⁺ est de 7 : 1 (mole : mole), de 8 : 1 (mole : mole), de 9 : 1 (mole : mole), de 10 : 1 (mole : mole), de 11:1 1 (mole : mole), ou de 12 : 1 (mole : mole).

6. Formulation pharmaceutique selon la revendication 1, dans laquelle le rapport Ca²⁺ sur Na⁺ est de 13 : 1 (mole : mole), de 14 : 1 (mole : mole), de 15 : 1 (mole : mole), ou de 16 : 1 (mole : mole).

7. Formulation pharmaceutique selon l'une quelconque des revendications 1 à 6, la formulation pharmaceutique étant une formulation liquide, dans laquelle, éventuellement, l'ion Ca²⁺ est présent en une concentration allant de 0,115 M à 1,15 M, ou dans laquelle l'ion Ca²⁺ est présent en une concentration allant de 0,575 M à 1,15 M.

8. Formulation pharmaceutique selon la revendication 7, dans laquelle l'ion Na⁺ est présent en une concentration allant de 0,053 M à 0,3 M, ou dans laquelle l'ion Na⁺ est présent en une concentration allant de 0,075 M à 0,3 M.

9. Formulation pharmaceutique selon la revendication 7 ou 8, dans laquelle le sel de calcium est choisi dans le groupe comprenant le chlorure de calcium, le carbonate de calcium, l'acétate de calcium, le phosphate de calcium, l'alginate de calcium, le stéarate de calcium, le sorbate de calcium, le sulfate de calcium, le gluconate de calcium, le lactate de calcium et le citrate de calcium, le sel de calcium étant éventuellement le lactate de calcium, le citrate de calcium, ou le sulfate de calcium, ou le sel de calcium étant le chlorure de calcium ou le lactate de calcium.

10. Formulation pharmaceutique selon l'une quelconque des revendications 7 à 9, dans laquelle le sel de sodium est choisi dans le groupe comprenant le chlorure de sodium, l'acétate de sodium, le bicarbonate de sodium, le carbonate de sodium, le sulfate de sodium, le stéarate de sodium, l'ascorbate de sodium, le benzoate de sodium, le biphosphate de sodium, le phosphate de sodium, le bisulfite de sodium, le citrate de sodium, le borate de sodium, le gluconate, de sodium, le métasilicate de sodium, et le lactate de sodium, le sel de sodium étant éventuellement le chlorure de sodium, ou le sel de sodium étant le lactate de sodium, le citrate de sodium, ou le sulfate de sodium.

11. Formulation pharmaceutique selon les revendications 1 à 6, la formulation pharmaceutique étant une formulation de poudre sèche.

12. Formulation pharmaceutique selon la revendication 11, dans laquelle le sel de calcium est présent en une quantité allant de 19,5 % à 90 % (p/p).

13. Formulation pharmaceutique selon la revendication 11 ou 12, dans laquelle le sel de calcium est choisi dans le groupe comprenant le chlorure de calcium, le carbonate de calcium, l'acétate de calcium, le phosphate de calcium, l'alginate de calcium, le stéarate de calcium, le sorbate de calcium, le sulfate de calcium, le gluconate de calcium, le lactate de calcium et le citrate de calcium, le sel de calcium étant éventuellement le lactate de calcium, le citrate de calcium, le sulfate de calcium, ou le chlorure de calcium.

14. Formulation pharmaceutique selon l'une quelconque des revendications 11 à 13, dans laquelle le sel de sodium est choisi dans le groupe comprenant le chlorure de sodium, l'acétate de sodium, le bicarbonate de sodium, le carbonate de sodium, le sulfate de sodium, le stéarate de sodium, l'ascorbate de sodium, le benzoate de sodium, le biphosphate de sodium, le phosphate de sodium, le bisulfite de sodium, le citrate de sodium, le borate de sodium, le gluconate de sodium, le métasilicate de sodium, et le lactate de sodium, le sel de sodium étant éventuellement le chlorure de sodium, ou le sel de sodium étant le lactate de sodium, le citrate de sodium, ou le sulfate de sodium.

15. Formulation pharmaceutique selon l'une quelconque des revendications 1 à 14, la formulation pharmaceutique étant formulée de sorte à administrer une dose de Ca²⁺ allant de 0,001 mg / kg de poids corporel / dose à 10 mg/kg de poids corporel / dose aux voies respiratoires, et/ou comprenant en outre un excipient, dans laquelle l'excipient est éventuellement choisi dans le groupe comprenant le lactose, la glycine, l'alanine, la leucine, l'isoleucine, le tréhalcse, la dipalmitoylphosphosphatidylcholine (DPPC), le diphosphatidyl-glycérol (DPPG), la 1,2-dipalmitoyl-sn-glycéro-3-phospho-L-sérine (DPPS), la 1,2-dipalmitoyl-sn-glycéro-3-phosphocholine (DSPC), la 1,2-distéaroyl-sn-glycéro-3-phosphoéthanolamine (DSPE), la 1-palmitoyl-2-cléoylphosphatidylcholine (POPC), le polyoxyéthylène-9-lauryl éther, le trioléate de sorbitane (Span 85), le glycocholate, la surfactine, le tyloxapol, le phosphate de sodium, le dextrane, la dextrine, le mannitol, la maltodextrine, l'albumine de sérum humain, l'albumine de sérum humain recombinée, et des polymères biodégradables, dans laquelle l'excipient est éventuellement la leucine, la maltodextrine, ou le mannitol.

16. Formulation pharmaceutique selon l'une quelconque des revendications 1 à 15, la formulation pharmaceutique étant une formulation de dose unitaire.

17. Quantité efficace de la formulation pharmaceutique selon l'une quelconque des revendications 1 à 16, destinée à être utilisée dans le traitement d'une infection des voies respiratoires chez un individu présentant une infection des voies respiratoires, manifestant les symptômes d'une infection des voies respiratoires, ou risquant de contracter une infection des voies respiratoires.

18. Quantité efficace de la formulation pharmaceutique selon l'une quelconque des revendications 1 à 16, destinée à être utilisée pour réduire la propagation d'une infection des voies respiratoires chez un individu présentant une infection des voies respiratoires, manifestant les symptômes d'une infection des voies respiratoires, ou risquant de contracter une infection des voies respiratoires.

19. Formulation pharmaceutique selon la revendication 17 ou 18, dans laquelle l'infection des voies respiratoires est la grippe, (i) la grippe étant éventuellement causée par le virus de la grippe A ou le virus de la grippe B, ou (ii) l'infection des voies respiratoires étant causée par un virus parainfluenza humain, le virus parainfluenza humain étant éventuellement un virus parainfluenza 3 humain (hPIV-3), ou (iii) l'infection des voies respiratoires étant causée par un rhinovirus, ou (iv) l'infection des voies respiratoires étant causée par un virus respiratoire syncytial (RSV), ou (v) dans laquelle l'infection des voies respiratoires est la pneumonie, la pneumonie étant éventuellement une pneumonie d'origine bactérienne, la pneumonie d'origine bactérienne étant éventuellement causée par *S. pneumoniae*, ou (vi) dans laquelle l'infection des voies respiratoires est une infection bactérienne, l'infection bactérienne étant éventuellement causée par une bactérie choisie dans le groupe comprenant *Streptococcus pneumoniae, Staphylococcus aureus, Streptococcus agalactiae, Streptococcus pyogenes, Haemophilus influenzae, Haemophilus parainfluenzae, Klebsiella pneumonia, Escherichia coli, Pseudomonas aeruginosa, Moraxella catarrhalis, Chlamydophila pneumoniae, Mycoplasma pneumoniae, Legionella pneumophila, Serratia marcescens, Burkholderia cepacia, Burkholderia pseudomallei, Bacillus anthracis, Bacillus cereus, Bordetella pertussis, Stenotrophomonas maltophilia,* une bactérie appartenant à la famille *citrobacter,* une bactérie appartenant à la famille *ecinetobacter,* et *mycobacterium tuberculosis.*

20. Quantité efficace de la formulation pharmaceutique selon l'une quelconque des revendications 1 à 16, destinée à être utilisée dans le traitement d'une maladie pulmonaire chez un individu ayant une maladie pulmonaire, présentant les symptômes d'une maladie pulmonaire, ou susceptible d'avoir une maladie pulmonaire.

21. Quantité efficace de la formulation pharmaceutique selon l'une quelconque des revendications 1 à 16, destinée à être utilisée dans le traitement d'une exacerbation aiguë d'une maladie pulmonaire chez un individu présentant une exacerbation aiguë d'une maladie pulmonaire, manifestant les symptômes d'une exacerbation aiguë d'une maladie pulmonaire, ou susceptible de présenter une exacerbation aiguë d'une maladie pulmonaire.

22. Formulation pharmaceutique selon les revendications 20 ou 21, la maladie pulmonaire étant l'asthme, l'hyperréactivité bronchique, la rhinite allergique, l'allergie allergique saisonnière, la bronchectasie, la bronchite chronique, l'emphysème, la maladie pulmonaire obstructive chronique, ou la mucoviscidose.

23. Formulation pharmaceutique selon la revendication 21, l'exacerbation aiguë d'une maladie pulmonaire chronique étant causée par une infection virale, une infection bactérienne, une infection fongique, une infection parasitaire, un allergène environnemental, ou un irritant environnemental.

24. Formulation pharmaceutique selon l'une quelconque des revendications 1 à 23, comprenant en outre un agent thérapeutique supplémentaire.
